# EUROPEAN PATENT APPLICATION

(11) **EP 3 443 977 A1**
(43) Date of publication of application: **20.02.2019**
(21) Application number: 18182135.6
(22) Date of filing: 30.12.2010
(51) Int. Cl.: A61K 38/07, A61P 9/10

(54) **METHODS FOR PERFORMING A CORONARY ARTERY BYPASS GRAFT PROCEDURE**

(30) Priority: 31.12.2009 US 29169909 P; 09.07.2010 US 36313810 P; 26.10.2010 US 40671310 P
(62) Divisional of application: 17174308.1
(71) Applicant: Stealth Peptides International, Inc., 98000 Monaco (MC)
(72) Inventor: BOROW, Kenneth, Bryn Mawr, PA Pennsylvania 19010 (US); WILSON, Travis D, Newton, MA Massachusetts 02461 (US)
(74) Representative: Barker Brettell LLP

(57) **Abstract**

The invention provides methods of treating an obstructive coronary artery disease in a mammalian subject. The methods comprise administering to the subject an effective amount of an aromatic-cationic peptide to subjects in need thereof, and performing a coronary artery bypass graft procedure on the subject.

## Description

### CROSS-REFERENCE TO RELATED APPLICATIONS

This application claims priority to U.S. Provisional Patent Application No. 61/291,699, filed December 31, 2009; U.S. Provisional Patent Application No. 61/363,138, filed July 9, 2010; and U.S. Provisional Patent Application No. 61/406,713, filed October 26, 2010, the entire contents of which are hereby incorporated by reference in their entirety.

### TECHNICAL FIELD

The present technology relates generally to compositions and methods for treating obstructive coronary artery disease using a coronary artery bypass graft (CABG) procedure. In particular, the methods relate to administering aromatic-cationic peptides in effective amounts prior to, during, and/or after a CABG procedure.

### BACKGROUND

The following description is provided to assist the understanding of the reader. None of the information provided or references cited is admitted to be prior art to the present invention.

Coronary artery bypass graft (CABG) surgery is effective in relieving angina and improving survival and quality of life in patients with obstructive coronary artery disease. It is among the most common operations performed in the world and accounts for more resources expended in cardiovascular medicine than any other single procedure. Indeed, in 2006, nearly 500,000 inpatient CABG procedures were performed in the United States. However, myocardial infarction, ventricular failure, life-threatening arrhythmias, renal insufficiency, neurological injury, and death can occur in the peri-operative and post-operative period. The incidence of such adverse events is expected to rise among patients referred for CABG, reflecting a patient population that is increasingly elderly and characterized by comorbid conditions, including advanced atherosclerosis.

Left ventricular (LV) function is an important predictor of early and late mortality after coronary artery surgery. It is associated with an increased risk of peri-operative and long-term mortality in patients undergoing coronary bypass surgery compared with patients with normal LV function. Both low ejection fraction (EF) and clinical heart failure are predictive of higher operative mortality rates with CABG surgery. Recently, it has been reported that postoperative NT-proBNP levels are associated with higher in-hospital mortality and prolonged ICU stay (>4 days) after CABG surgery.

Any increase in creatinine kinase-MB fraction (CK-MB) after CABG surgery is suggestive of myocyte necrosis, and higher levels of CK-MB are likely to be associated with worse outcomes. A linear relation between post-operative CK-MB elevation and mortality has been reported with post-operative peak CK-MB values of <5 times, 5 to <10 times, 10 to <20 times, and >20 times the upper limit of normal associated with 3.4%, 5.8%, 7.8%, and 20.2% six month mortality, respectively. A recent consensus document recommended a definition of myocardial infarction following CABG surgery based on a CK-MB elevation of at least 5 times the upper limit of normal during the first 72 hours following CABG surgery associated with the appearance of new pathological Q waves or left bundle-branch block, angiographically documented new graft or native coronary artery occlusion, or imaging evidence of new loss of viable myocardium.

Ultimately, if outcomes among CABG patients are to be improved, the development of better means for preventing myocardial ischemia-reperfusion injury, an important mechanism underlying the increased cardiovascular morbidity and mortality, will be important. In addition, developing and targeting novel adjunctive therapies to mitigate or minimize injury to other vulnerable end-organs (*e*.*g*., kidney and brain) remain important to improving outcomes in patients undergoing CABG.

### SUMMARY

The present technology relates generally to the treatment of obstructive coronary artery disease in mammals through administration of therapeutically effective amounts of aromatic-cationic peptides to subjects in need thereof. In one aspect, the present disclosure provides a method of treating obstructive coronary artery disease comprising: (a) administering to a mammalian subject in need thereof a therapeutically effective amount of the peptide D-Arg-2'6'-Dmt-Lys-Phe-NH₂ or a pharmaceutically acceptable salt thereof; and (b) performing a coronary artery bypass graft procedure on the subject. In another aspect, the present disclosure provides a method for preventing renal or cerebral complications during a coronary artery bypass graft procedure (CABG) procedure, the method comprising: (a) administering to a mammalian subject a therapeutically effective amount of the peptide D-Arg-2'6'-Dmt-Lys-Phe-NH₂ or a pharmaceutically acceptable salt thereof; and (b) performing a coronary artery bypass graft procedure (CABG) on the subject.

In some embodiments, the aromatic-cationic peptide is a peptide having:
at least one net positive charge;
a minimum of four amino acids;
a maximum of about twenty amino acids;
a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1. In one embodiment, 2pₘ is the largest number that is less than or equal to r+1, and a may be equal to pₜ. The aromatic-cationic peptide may be a water-soluble peptide having a minimum of two or a minimum of three positive charges.

In one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a maximum of about 6, a maximum of about 9, or a maximum of about 12 amino acids.

In one embodiment, the peptide comprises a tyrosine or a 2',6'-dimethyltyrosine (Dmt) residue at the N-terminus. For example, the peptide may have the formula Tyr-D-Arg-Phe-Lys-NH₂ or 2',6'-Dmt-D-Arg-Phe-Lys-NH₂. In another embodiment, the peptide comprises a phenylalanine or a 2',6'-dimethylphenylalanine residue at the N-terminus. For example, the peptide may have the formula Phe-D-Arg-Phe-Lys-NH₂ or 2',6'-Dmp-D-Arg-Phe-Lys-NH₂. In a particular embodiment, the aromatic-cationic peptide has the formula D-Arg-2',6'-Dmt-Lys-Phe-NH₂ (also known as SS-31).

In one embodiment, the peptide is defined by formula I: 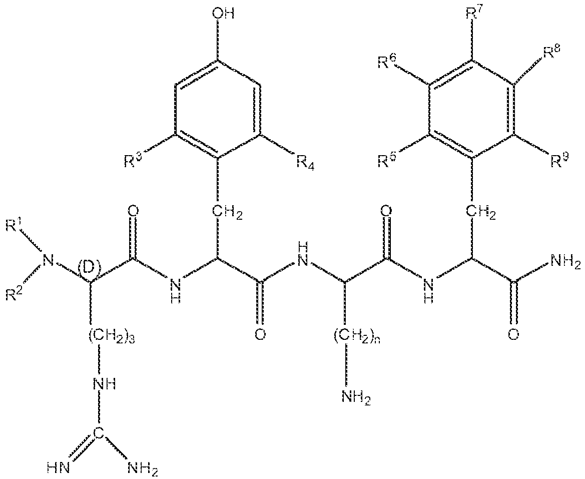
wherein R¹ and R² are each independently selected from
   (i) hydrogen;
   (ii) linear or branched C₁-C₆ alkyl;
   (iii) 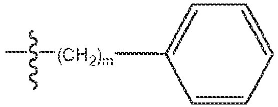 where m = 1-3;
   (iv) 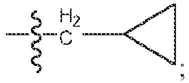
   (v) 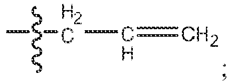
R³ and R⁴ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo;
R⁵, R⁶, R⁷, R⁸, and R⁹ are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and n is an integer from 1 to 5.

In a particular embodiment, R¹ and R² are hydrogen; R³ and R⁴ are methyl; R⁵, R⁶, R⁷, R⁸, and R⁹ are all hydrogen; and n is 4.

In one embodiment, the peptide is defined by formula II: 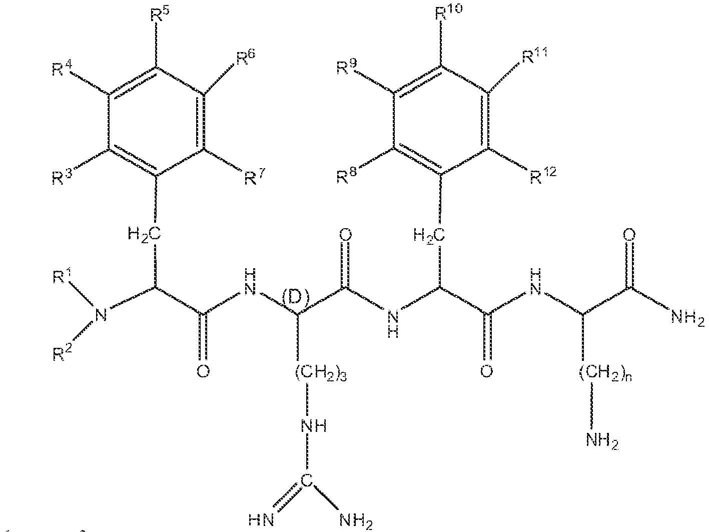
wherein R¹ and R² are each independently selected from
   (i) hydrogen;
   (ii) linear or branched C₁-C₆ alkyl;
   (iii) 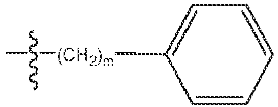 where m = 1-3;
   (iv) 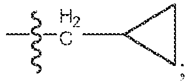
   (v) 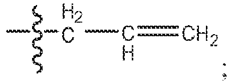
R³, R⁴, R⁵, R⁶, R⁷, R⁸, R⁹, R¹⁰, R¹¹ and R¹² are each independently selected from
   (i) hydrogen;
   (ii) linear or branched C₁-C₆ alkyl;
   (iii) C₁-C₆ alkoxy;
   (iv) amino;
   (v) C₁-C₄ alkylamino;
   (vi) C₁-C₄ dialkylamino;
   (vii) nitro;
   (viii) hydroxyl;
   (ix) halogen, where "halogen" encompasses chloro, fluoro, bromo, and iodo; and n is an integer from 1 to 5.

In a particular embodiment, R¹, R², R³, R⁴, R⁵, R⁶, R⁷, R⁸, R⁹, R¹⁰, R¹¹, and R¹² are all hydrogen; and n is 4. In another embodiment, R¹, R², R³, R⁴, R⁵, R⁶, R⁷, R⁸, R⁹, and R¹¹ are all hydrogen; R⁸ and R¹² are methyl; R¹⁰ is hydroxyl; and n is 4.

The aromatic-cationic peptides may be administered in a variety of ways. In some embodiments, the peptides may be administered orally, topically, intranasally, intraperitoneally, intravenously, subcutaneously, or transdermally (*e*.*g*., by iontophoresis).

In one embodiment, the subject is administered the peptide prior to ischemia. In one embodiment, the subject is administered the peptide prior to the reperfusion of ischemic tissue. In one embodiment, the subject is administered the peptide at about the time of reperfusion of ischemic tissue. In one embodiment, the subject is administered the peptide after reperfusion of ischemic tissue.

In one embodiment, the subject is administered the peptide prior to the CABG procedure. In another embodiment, the subject is administered the peptide after the CABG procedure. In another embodiment, the subject is administered the peptide during and after the CABG procedure. In yet another embodiment, the subject is administered the peptide continuously before, during, and after the CABG procedure.

In one embodiment, the subject is administered the peptide starting at least 5 minutes, at least 10 min, at least 30 min, at least 1 hour, at least 3 hours, at least 5 hours, at least 8 hours, at least 12 hours, or at least 24 hours prior to CABG. In one embodiment, the subject is administered the peptide starting at about 5-30 min, from about 10-60 minutes, from about 10-90 min, or from about 10-120 min prior to the CABG procedure. In one embodiment, the subject is administered the peptide until about 5-30 min, until about 10-60 min, until about 10-90 min, until about 10-120 min, or until about 10-180 min after the CABG procedure.

In one embodiment, the subject is administered the peptide for at least 30 min, at least 1 hour, at least 3 hours, at least 5 hours, at least 8 hours, at least 12 hours, or at least 24 hours after the CABG procedure tissue. In one embodiment, the duration of administration of the peptide is about 30 min, about 1 hour, about 2 hours, about 3 hours, about 4 hours, about 5 hours, about 8 hours, about 12 hours, or about 24 hours after the CABG procedure.

In one embodiment, the subject is administered the peptide as an IV infusion starting at about 1 min to 30 min prior to reperfusion (*i.e.* about 5 min, about 10 min, about 20 min, or about 30 min prior to reperfusion) and continuing for about 1 hour to 24 hours after reperfusion (*i*.*e*., about 1 hour, about 2 hours, about 3 hours, or about 4 hours after reperfusion). In one embodiment, the subject receives in IV bolus injection prior to reperfusion of the tissue. In one embodiment, the subject continues to receive the peptide chronically after the reperfusion period, *i.e.* for about 1-7 days, about 1-14 days, about 1-30 days after the reperfusion period. During this period, the peptide may be administered by any route, e.g., subcutaneously or intravenously.

In one embodiment, the peptide is administered by a systemic intravenous infusion commencing about 5-60, about 10-45, or about 30 minutes before the induction of anesthesia. In one embodiment, the peptide is administered in conjunction with a cardioplegia solution. In one embodiment, the peptide is administered as part of the priming solution in a heart lung machine during cardiopulmonary bypass.

Aspects of the invention may also be provided by the following numbered paragraphs
1. A method for treating obstructive coronary artery disease comprising:
   (a) administering to a mammalian subject a therapeutically effective amount of the peptide D-Arg-2'6'-Dmt-Lys-Phe-NH₂ or a pharmaceutically acceptable salt thereof; and
   (b) performing a coronary artery bypass graft procedure (CABG) on the subject.
2. The method of paragraph 1, wherein the subject is administered the peptide prior to the CABG procedure.
3. The method of paragraph 1, wherein the subject is administered the peptide after the CABG procedure.
4. The method of paragraph 1, wherein the subject is administered the peptide during and after the CABG procedure.
5. The method of paragraph 1, wherein the subject is administered the peptide continuously before, during, and after the CABG procedure.
6. The method of paragraph 5, wherein the subject is administered the peptide for at least 3 hours after the CABG procedure.
7. The method of paragraph 5, wherein the subject is administered the peptide for at least 5 hours after the CABG procedure.
8. The method of paragraph 5, wherein the subject is administered the peptide for at least 8 hours after the CABG procedure.
9. The method of paragraph 5, wherein the subject is administered the peptide for at least 12 hours after the CABG procedure.
10. The method of paragraph 5, wherein the subject is administered the peptide for at least 24 hours after the CABG procedure.
11. The method of paragraph 5, wherein the subject is administered the peptide starting at least 8 hours before the CABG procedure.
12. The method of paragraph 5, wherein the subject is administered the peptide starting at least 5 hours before the CABG procedure.
13. The method of paragraph 5, wherein the subject is administered the peptide starting at least 2 hours before the CABG procedure.
14. The method of paragraph 5, wherein the subject is administered the peptide starting at least 1 hour before the CABG procedure.
15. The method of paragraph 5, wherein the subject is administered the peptide starting at least 30 minutes before the CABG procedure.
16. The method of paragraph 1, wherein the peptide is administered by a systemic intravenous infusion commencing about 30 minutes before the induction of anesthesia.
17. The method of paragraph 1, wherein the peptide is administered in conjunction with a cardioplegia solution.
18. The method of paragraph 1, wherein the peptide is administered as part of the priming solution in a heart lung machine during cardiopulmonary bypass.
19. The method of paragraph 1, wherein the levels of one or more of N-terminal pro-brain natriuretic peptide (NT-proBNP), glucose, and estimated glomerular filtration rate (eGFR) are reduced in a subject administered the peptide relative to a comparable subject undergoing a CABG procedure, but not administered the peptide.
20. A method for preventing renal or cerebral complications during a coronary artery bypass graft procedure (CABG) procedure, the method comprising:
   (a) administering to a mammalian subject a therapeutically effective amount of the peptide D-Arg-2'6'-Dmt-Lys-Phe-NH₂ or a pharmaceutically acceptable salt thereof; and
   (b) performing a coronary artery bypass graft procedure (CABG) on the subject.

### BRIEF DESCRIPTION OF THE FIGURES

FIG. 1 is an illustration of the study design for animals used in the examples.
FIGs. 2A and 2B present data showing infarct size for rabbits with a sham treatment (ligature applied, but not tightened). FIG. 2A is a photograph of heart slices and a computer-generated image highlighting infarct size of a sham rabbit treated with a placebo. FIG. 2B is a photograph of heart slices and a computer-generated image highlighting infarct size of a sham rabbit treated with peptide.
FIGs. 3A and 3B present data showing infarct size for two different control rabbits with induced cardiac ischemia and treated with a placebo. Each figure shows a photograph of heart slices and a computer-generated image highlighting infarct size.
FIGs. 4A, 4B, 4C, 4D, and 4E present data showing infarct size for five different rabbits with induced cardiac ischemia and treated with an illustrative aromatic-cationic peptide. Each figure shows a photograph of heart slices and a computer-generated image highlighting infarct size.
FIG. 5 is a chart showing the ratio of infracted area to left ventricular area for each of the control and test groups of rabbits.
FIG. 6 is a chart showing the ratio of infracted area to area of risk for each of the control and test groups of rabbits.

### DETAILED DESCRIPTION

It is to be appreciated that certain aspects, modes, embodiments, variations and features of the invention are described below in various levels of detail in order to provide a substantial understanding of the present invention.

The definitions of certain terms as used in this specification are provided below. Unless defined otherwise, all technical and scientific terms used herein generally have the same meaning as commonly understood by one of ordinary skill in the art to which this invention belongs.

As used in this specification and the appended claims, the singular forms "a," "an" and "the" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, intranasally, parenterally (intravenously, intramuscularly, intraperitoneally, or subcutaneously), or topically. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e*.*g*., hydroxyproline, γ-carboxyglutamate, and O-phosphoserine. Amino acid analogs refers to compounds that have the same basic chemical structure as a naturally-occurring amino acid, *i.e*., an α-carbon that is bound to a hydrogen, a carboxyl group, an amino group, and an R group, *e*.*g*., homoserine, norleucine, methionine sulfoxide, methionine methyl sulfonium. Such analogs have modified R groups (*e*.*g*., norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect, *e*.*g*., an amount which results in the prevention of, or a decrease in, cardiac ischemia-reperfusion injury or one or more symptoms associated with cardiac ischemia-reperfusion injury. In the context of therapeutic or prophylactic applications, the amount of a composition administered to the subject will depend on the type and severity of the disease and on the characteristics of the individual, such as general health, age, sex, body weight and tolerance to drugs. It will also depend on the degree, severity and type of disease. The skilled artisan will be able to determine appropriate dosages depending on these and other factors. The compositions can also be administered in combination with one or more additional therapeutic compounds. In the methods described herein, the aromatic-cationic peptides may be administered to a subject having one or more signs or symptoms of vessel occlusion. In other embodiments, the mammal has one or more signs or symptoms of myocardial infarction, such as chest pain described as a pressure sensation, fullness, or squeezing in the mid portion of the thorax; radiation of chest pain into the jaw or teeth, shoulder, arm, and/or back; dyspnea or shortness of breath; epigastric discomfort with or without nausea and vomiting; and diaphoresis or sweating. For example, a "therapeutically effective amount" of the aromatic-cationic peptides is meant levels in which the physiological effects of a cardiac ischemia-reperfusion injury during a CABG procedure are, at a minimum, ameliorated.

As used herein the term "ischemia reperfusion injury" refers to the damage caused first by restriction of the blood supply to a tissue followed by a sudden resupply of blood and the attendant generation of free radicals. Ischemia is a decrease in the blood supply to the tissue and is followed by reperfusion, a sudden perfusion of oxygen into the deprived tissue.

An "isolated" or "purified" polypeptide or peptide is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the terms "polypeptide," "peptide," and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, the term "simultaneous" therapeutic use refers to the administration of at least two active ingredients by the same route and at the same time or at substantially the same time.

As used herein, the term "separate" therapeutic use refers to an administration of at least two active ingredients at the same time or at substantially the same time by different routes.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

As used herein, the terms "treating" or "treatment" or "alleviation" refers to both therapeutic treatment and prophylactic or preventative measures, wherein the object is to prevent or slow down (lessen) the targeted pathologic condition or disorder. A subject is successfully "treated" for vessel occlusion injury if, after receiving a therapeutic amount of the aromatic-cationic peptides according to the methods described herein, the subject shows observable and/or measurable reduction in or absence of one or more signs and symptoms of vessel occlusion injury, such as, *e*.*g*., reduced infarct size. It is also to be appreciated that the various modes of treatment or prevention of medical conditions as described are intended to mean "substantial," which includes total but also less than total treatment or prevention, and wherein some biologically or medically relevant result is achieved.

As used herein, "prevention" or "preventing" of a disorder or condition refers to a compound that reduces the occurrence of the disorder or condition in the treated sample relative to an untreated control sample, or delays the onset or reduces the severity of one or more symptoms of the disorder or condition relative to the untreated control sample. As used herein, preventing renal or cerebral complications of CABG includes preventing or ameliorating damage to the brain or kidneys in a statistical sample in a patient undergoing CABG. Preventing does not mean that a subject never develops the condition later in life - only that the probability of occurrence is reduced.

### Methods of Performing a CABG Procedure with Aromatic-Cationic Peptides

The present technology relates to the treatment or prevention of obstructive coronary artery disease by administration of certain aromatic-cationic peptides in conjunction with a CABG procedure. Also provided is a method for the treatment or prevention of cardiac ischemia-reperfusion injury. In one aspect, the present technology relates to a method of coronary revascularization comprising administering to a mammalian subject a therapeutically effective amount of the aromatic cationic peptide and performing a coronary artery bypass graft (CABG) procedure on the subject.

Certain aromatic cationic peptides, including D-Arg-2',6'-Dmt-Lys-Phe-NH₂, have been shown to be beneficial in a wide variety of *in vivo* animal models of myocardial ischemia reperfusion (IR) injury. In an acute myocardial IR model, rabbits were subjected to 30 minutes of ischemia followed by 180 minutes of reperfusion. Infusion of D-Arg-2',6'-Dmt-Lys-Phe-NH2 or vehicle was started 20 minutes prior to reperfusion and continued through the experiment. The infarct size was determined by calculating the ratio of the amount of the LV at risk for infarction (measured by the amount of area that did not take up Evans Blue dye on histology) to the infarct area (measured by the amount of area that did not stain with triphenyl-tetrazolium-chloride).

In one aspect, the present disclosure relates to methods for using an aromatic-cationic peptide as a multi-organ protectant when administered prior to, during, and immediately post-surgery in patients who are undergoing a CABG procedure. Cardiopulmonary bypass is known to induce oxidative stress. During reoxygenation and reperfusion of ischemic myocardium, oxygen-derived free radicals (superoxide anion, hydroxyl anion, and hydrogen peroxide) are produced and the normal endogenous mechanism of scavenging these free radicals is reduced. In most cases, these oxygen radicals are by-products of cellular metabolism and are scavenged and deactivated enzymatically by superoxide dismutase, catalase, and peroxidase and by antioxidant receptors such as glutathione, vitamin E, and hemoglobin. Excess production of reactive oxygen species (ROS) during reperfusion of myocardium is damaging to cellular membranes and allows enzyme leakage into the tissue interstitium resulting in depletion of superoxide dismutase and catalase.

The mitochondria are the primary intracellular source of ROS. Functionally, mitochondria are both the initiator and the first target of oxidative stress. Mitochondrial damage can lead to cell death. This reflects the critical role that mitochondria play in energy metabolism and calcium homeostasis as well as the ability of mitochondria to release pro-apoptotic factors such as cytochrome C and apoptosis-inducing factor. Mitochondria are very sensitive to ischemia. Indeed, mitochondrial damage and dysfunction can occur even after periods of moderately reduced myocardial blood flow without immediate changes in levels of ATP or phosphocreatine.

Reperfusion injury is related, at least in part, to problems with mitochondrial permeability transition. Ultimately, this results in generation of low ATP concentrations and altered ion homeostasis leading to rupture of plasma membranes and cell death. Post reperfusion arrhythmias have also been associated with mitochondrial dysfunction. Previous attempts to individually target known mediators of ischemia-reperfusion injury in patients using antioxidant therapy, calcium-channel blockers, sodium-hydrogen exchange inhibitors, and anti-inflammatory drugs have been largely disappointing. This has led to the concept that multi-targeted mechanistic approaches to ischemia-reperfusion injury are required to successfully translate experimental interventions into protection against the clinical manifestations of reperfusion injury which include: reperfusion arrhythmias, myocardial stunning, and myocyte death and infarction.

Such a broad-based approach towards myocardial salvage at the cellular level in CABG surgery must include therapies that prevent ischemia-reperfusion injury while maintaining blood flow throughout the myocardial microcirculation. In theory, this is best accomplished by integrating technically well-performed CABG surgery and therapeutic agents that can accomplish the dual goal of time-critical opening of large conduit arteries and maintenance of open microvasculature. Unfortunately, as a result of the multiple cardiac pathophysiologic derangements encountered with CABG surgery, effective therapies to reduce or prevent CABG associated myocardial ischemia-reperfusion injury have proven elusive.

Certain aromatic-cationic peptides are capable of mitochondrial targeting. Uptake studies showed that the intracellular concentration of the peptide D-Arg-2',6'-Dmt-Lys-Phe-NH₂ is six-fold higher than in the extracellular fluid and the concentration of the drug in the mitochondrial pellet is approximately 5000-fold higher. Thus, this peptide is selectively taken up by mitochondria. In conjunction with this localization in mitochondria, this peptide has been shown to have multiple unique characteristics including: scavenging reactive oxygen species (ROS); facilitating electron transfer within the mitochondrial electron transport chain; maintaining mitochondrial respiration (oxygen consumption); maintaining adenosine triphosphate (ATP) levels; preventing loss of mitochondrial membrane potential; preventing release of cytochrome c; and preventing mitochondrial swelling consistent with inhibition of the mitochondrial permeability transition pore (mPTP) opening.

In some embodiments, the administration of an aromatic-cationic peptide prior to, during, and/or immediately after a coronary artery bypass graft procedure prevents or treats renal complications of CABG surgery. In post-operative CABG patients, even minor increases in serum creatinine above baseline values are associated with adverse outcomes and any degree of renal insufficiency, no matter how small, has significant clinical consequences even in the absence of complete loss of function. Peri-operative insults including ischemia-reperfusion injury may result in the development of renal injury that is manifested by a decrease in glomerular filtration rate (GFR) and a rise in serum creatinine concentration. Despite advances in cardiopulmonary bypass techniques, intensive unit care, and hemodialysis, morbidity and mortality associated with post-operative renal dysfunction have not changed significantly over the past decade. While different intraoperative strategies have been developed to provide renal protection in patients undergoing cardiovascular procedures, these strategies have focused mainly on the use of drugs such as dopamine, mannitol, and furosemide. However, no pharmacological intervention has proven to be renal protective.

The peptide D-Arg-2',6'-Dmt-Lys-Phe-NH₂ has been shown to be effective in reducing the incidence of ARI caused by ischemia-reperfusion. See U.S. Patent Publication No. 20090221514. In particular, this peptide is effective in reducing interstitial fibrosis, tubular apoptosis, macrophage infiltration and tubular proliferation in a animal model of ARI. The peptide significantly improved histopathological score resulting from 45 min ischemia and 24 h reperfusion, and also significantly increased rate of ATP production after reperfusion.

Risk factors associated with renal dysfunction in post-operative CABG patients can be divided into patient-related and procedure-related criteria. Patient-related factors include diabetes mellitus, hypertension, left ventricular dysfunction, and preexisting kidney disease. For example, patients with preoperative renal serum creatinine (SCr) ≥1.5 mg/dL compared to subjects with lower values are at greater risk for acute worsening of post-operative renal function, prolonged mechanical ventilation, increased intensive care unit and hospital stays, and greater short- and long-term mortality. For non-dialysis patients with pre-operative SCr ≥1.7 and <2.5 mg/dL, the peri-operative mortality is incrementally increased and may be as high as 33%. Overall, subjects who pre-operatively have a reduced number of normally functioning nephrons are more vulnerable during the peri- and post-operative period to maldistributed and decreased renal blood flow, increased renal vascular resistance, and decreased glomerular filtration rate.

Preexisting kidney disease greatly increases the risk of peri-operative complications. Renal function declines with age and dysfunction is a consequence of several conventional cardiovascular risk factors such as hypertension and diabetes. Impaired kidney function exacerbates the effects of these conditions and is associated with a variety of other less well defined risk factors including increased acute phase proteins, reduced antioxidants, and deranged calcium/phosphate metabolism. Renal dysfunction is also a common consequence of reduced left ventricular systolic function and heart failure. Likewise, chronic kidney disease is itself a risk factor for left ventricular hypertrophy, dilatation, and dysfunction.

The working definition of acute kidney injury (AKI) requires an abrupt (within 48 hours) reduction in kidney function defined as an absolute increase in serum creatinine level of ≥ 26.4 µmol/l (0.3 mg/dl) OR a percentage increase in serum creatinine level of ≥ 50% (1.5 fold higher than baseline) OR a reduction in urine output (documented oliguria of <0.5 ml/kg/h for >6 h). It is assumed that these criteria are applied in the context of the clinical presentation and following adequate fluid resuscitation when applicable. Overall, the AKIN proposed three classes describing increases in serum creatinine relative to baseline as well as decreases in post-operative urine output.

Multiple general pathophysiologic processes are thought to contribute to CABG surgery associated AKI. Included in this list are ischemia-reperfusion injury, oxidative stress and inflammation. These factors in particular appear to act in an interrelated and probably synergistic manner. Normally, kidney perfusion is autoregulated such that glomerular filtration rate is maintained until the mean arterial blood pressure falls below 80 mm Hg. Mean arterial blood pressure during cardiac surgery is often at the lower limits or below the limits of autoregulation, especially during periods of hemodynamic instability. In addition, many cardiac surgery patients have impaired autoregulation due to existing comorbidities (*e*.*g*., advanced age, atherosclerosis, chronic hypertension, or chronic kidney disease), administration of drugs that impact kidney autoregulation (*e*.*g*., nonsteroidal anti-inflammatory drugs, ACE inhibitors, angiotensin receptor blockers, and radiocontrast agents), or a proinflammatory state. In patients with impaired autoregulation, kidney function may deteriorate even when the mean arterial blood pressure is within the normal range.

In CABG patients, these factors can result in cellular ischemia with tubular epithelial and vascular endothelial injury and activation. In addition, microvascular as well as tubular obstruction can occur leading to a worsening cycle of injury and cell loss. Injury is then either stabilized during a maintenance phase when cellular repair, division, and redifferentiation take place and transitions to the recovery phase or persistent release of injurious mediators drive cellular responses toward inappropriate proliferation and fibrosis. Finally, nucleotide depletion culminates in the accumulation of hypoxanthine and contributes to the generation of reactive oxygen molecules. Tubular oxidative stress is evident even in off-pump cardiac surgery and is exacerbated by cardiopulmonary bypass. Current evidence suggests that apoptosis is the prime mechanism of early tubular cell death in AKI. The key step in apoptosis is activation of caspases (cysteine aspartate-specific proteinases) which are highly operative during programmed cell death. The activation of caspases occurs by pathways that govern mitochondrial membrane permeability that induce pores in the mitochondria allowing cytochrome-c to egress into the cytoplasm which then activates the caspase cascade.

Cardiac surgery can also contribute to ischemic kidney injury by inciting a strong systemic inflammatory response. Proinflammatory events during cardiac surgery include operative trauma, contact of the blood components with the artificial surface of the CPB circuit, ischemia-reperfusion injury, and endotoxemia. This systemic inflammatory reaction can result in dysfunction of multiple end-organs including the kidneys, lungs, heart, and brain.

The realization that CABG associated AKI is a complex interplay among ischemia, endothelial dysfunction, and tubular injury has led to the search for alternative renal protective approaches. The ability of certain aromatic-cationic to target all of these processes as well as beneficially impact multiple sites upstream and at the termination of both tubular and vascular injury has led to the discovery of this molecule as a renal-protective agent.

In some embodiments, the administration of an aromatic-cationic peptide prior to, during, and/or immediately after a CABG procedure prevents or treats cerebral complications of CABG surgery. Post-operative neurologic deterioration has been reported in patients undergoing CABG surgery, especially when CPB is used. *See* Terrando *et al.,* Tumor necrosiss factor-α triggers a cytokine cascade yielding postoperative cognitive decline. Proc Natl Acad Sci USA 107(47): 20518-20522,2010. Despite the many advances made in cardiac surgery, peri- and post-operative cerebral injury remains a problem. Reduced perfusion pressure during CPB as well as embolization of air or particulate matter during aortic cannulation or weaning from CPB may produce neurologic damage and neuropsychiatric complications.

The peptide D-Arg-2',6'-Dmt-Lys-Phe-NH₂ has been shown to protect mice from cerebral ischemia. See U.S. Patent Publication No. 20070129306. Treatment of wild type mice with this peptide at 0, 6, 24 and 48 hours after 30 min occlusion of the middle cerebral artery resulted in a significant reduction in infarct volume and hemispheric swelling compared to saline controls.

It has been established that CPB can cause a systemic inflammatory response which may contribute to the development of neurologic injury in patients undergoing CABG surgery. This systemic inflammation may be mediated by surgical trauma, blood contact with the extracorporeal bypass circuit, and lung reperfusion injury after the discontinuation of CPB. The CBP related systemic inflammatory response correlates with serum C-reactive protein, IL-6, IL-8, and cortisol concentrations. Attenuating this systemic inflammatory response is an important therapeutic objective and is associated with improved outcome.

In addition, local cerebral events can be detrimental to the patient during cardiac surgery. The presence of cerebral ischemia itself induces a complex series of molecular pathways involving signaling mechanisms, gene transcription, and protein formation. Within seconds to minutes after the loss of blood flow to a region of the brain, the ischemic cascade is initiated leading to a series of biochemical events that eventually result in disintegration of cell membranes and neuronal death at the center/core of the infarction. Associated with these events are the concerted action of multiple pathogenic effectors including oxidative stress, ATP depletion, excitotoxicity, inflammation, apoptosis, microvascular obstruction and disruption of the brain's blood-brain barrier. Oxidative stress leads to ischemic cell death that involves the formation of ROS/reactive nitrogen species through multiple injury mechanisms including mitochondrial inhibition, Ca²⁺ overload, and ischemia-reperfusion injury. Ischemic injury induced local inflammation is caused by activated microglia and infiltrated inflammatory cells that further release pro-inflammatory cytokines and ROS within the injured site. Since brain tissue is not well equipped with antioxidant and anti-inflammatory defenses, these processes threaten the viability of ischemic cerebral tissue. Cerebral ischemia-reperfusion induces a significant shift toward a pro-oxidative status in the brain. For this reason, the peptide D-Arg-2',6'-Dmt-Lys-Phe-NH₂ may be used as a neuro protectant during a CABG procedure.

In one embodiment, the subject is administered the peptide during and after the CABG procedure. In another embodiment, the subject is administered the peptide continuously before, during, and the CABG procedure. In one embodiment, the subject is administered the peptide starting at least 10 min, at least 30 min, at least 1 hour, at least 3 hours, at least 5 hours, at least 8 hours, at least 12 hours, or at least 24 hours prior to the CABG procedure. In one embodiment, the subject is administered the peptide for at least 3 hours, at least 5 hours, at least 8 hours, at least 12 hours, or at least 24 hours after the CABG procedure. In one embodiment, the subject is administered the peptide starting at least 8 hours, at least 4 hours, at least 2 hours, at least 1 hour, or at least 30 minutes prior to the CABG procedure. In one embodiment, the subject is administered for at least one week, at least one month or at least one year after the CABG procedure.

Aromatic-cationic peptides are water-soluble and highly polar. Despite these properties, the peptides can readily penetrate cell membranes. The aromatic-cationic peptides typically include a minimum of three amino acids or a minimum of four amino acids, covalently joined by peptide bonds. The maximum number of amino acids present in the aromatic-cationic peptides is about twenty amino acids covalently joined by peptide bonds. Suitably, the maximum number of amino acids is about twelve, more preferably about nine, and most preferably about six.

The amino acids of the aromatic-cationic peptides can be any amino acid. As used herein, the term "amino acid" is used to refer to any organic molecule that contains at least one amino group and at least one carboxyl group. Typically, at least one amino group is at the α position relative to a carboxyl group. The amino acids may be naturally occurring. Naturally occurring amino acids include, for example, the twenty most common levorotatory (L) amino acids normally found in mammalian proteins, *i.e.,* alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val). Other naturally occurring amino acids include, for example, amino acids that are synthesized in metabolic processes not associated with protein synthesis. For example, the amino acids ornithine and citrulline are synthesized in mammalian metabolism during the production of urea. Another example of a naturally occurring amino acid includes hydroxyproline (Hyp).

The peptides optionally contain one or more non-naturally occurring amino acids. Optimally, the peptide has no amino acids that are naturally occurring. The non-naturally occurring amino acids may be levorotary (L-), dextrorotatory (D-), or mixtures thereof. Non-naturally occurring amino acids are those amino acids that typically are not synthesized in normal metabolic processes in living organisms, and do not naturally occur in proteins. In addition, the non-naturally occurring amino acids suitably are also not recognized by common proteases. The non-naturally occurring amino acid can be present at any position in the peptide. For example, the non-naturally occurring amino acid can be at the N-terminus, the C-terminus, or at any position between the N-terminus and the C-terminus.

The non-natural amino acids may, for example, comprise alkyl, aryl, or alkylaryl groups not found in natural amino acids. Some examples of non-natural alkyl amino acids include α-aminobutyric acid, β-aminobutyric acid, γ-aminobutyric acid, δ-aminovaleric acid, and ε-aminocaproic acid. Some examples of non-natural aryl amino acids include ortho-, meta, and para-aminobenzoic acid. Some examples of non-natural alkylaryl amino acids include ortho-, meta-, and para-aminophenylacetic acid, and γ-phenyl-β-aminobutyric acid. Non-naturally occurring amino acids include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy (*i.e*., alkoxy), amino, C₁-C₄ alkylamino and C₁-C₄ dialkylamino (*e.g.,* methylamino, dimethylamino), nitro, hydroxyl, halo (*i.e.,* fluoro, chloro, bromo, or iodo). Some specific examples of non-naturally occurring derivatives of naturally occurring amino acids include norvaline (Nva) and norleucine (Nle).

Another example of a modification of an amino acid in a peptide is the derivatization of a carboxyl group of an aspartic acid or a glutamic acid residue of the peptide. One example of derivatization is amidation with ammonia or with a primary or secondary amine, *e*.*g*. methylamine, ethylamine, dimethylamine or diethylamine. Another example of derivatization includes esterification with, for example, methyl or ethyl alcohol. Another such modification includes derivatization of an amino group of a lysine, arginine, or histidine residue. For example, such amino groups can be acylated. Some suitable acyl groups include, for example, a benzoyl group or an alkanoyl group comprising any of the C₁-C₄ alkyl groups mentioned above, such as an acetyl or propionyl group.

The non-naturally occurring amino acids are suitably resistant, and/or insensitive to common proteases. Examples of non-naturally occurring amino acids that are resistant or insensitive to proteases include the dextrorotatory (D-) form of any of the above-mentioned naturally occurring L-amino acids, as well as L- and/or D- non-naturally occurring amino acids. The D-amino acids do not normally occur in proteins, although they are found in certain peptide antibiotics that are synthesized by means other than the normal ribosomal protein synthetic machinery of the cell. As used herein, the D-amino acids are considered to be non-naturally occurring amino acids.

In order to minimize protease sensitivity, the peptides should have less than five, less than four, less than three, or less than two contiguous L-amino acids recognized by common proteases, irrespective of whether the amino acids are naturally or non-naturally occurring. Optimally, the peptide has only D-amino acids, and no L-amino acids. If the peptide contains protease sensitive sequences of amino acids, at least one of the amino acids is preferably a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine.

The aromatic-cationic peptides should have a minimum number of net positive charges at physiological pH in comparison to the total number of amino acid residues in the peptide. The minimum number of net positive charges at physiological pH will be referred to below as (pₘ). The total number of amino acid residues in the peptide will be referred to below as (r). The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

"Net charge" as used herein refers to the balance of the number of positive charges and the number of negative charges carried by the amino acids present in the peptide. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-D-Arg has one negatively charged amino acid (*i.e*., Glu) and four positively charged amino acids (*i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

**TABLE 1. Amino acid number and net positive charges (3pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

**TABLE 2. Amino acid number and net positive charges (2pₘ ≤ p+1)**

| | | | | | | | | | | | | | | | | | | |
|---|---|---|---|---|---|---|---|---|---|---|---|---|---|---|---|---|---|---|
| **(r)** | 3 | 4 | 5 | 6 | 7 | 8 | 9 | 10 | 11 | 12 | 13 | 14 | 15 | 16 | 17 | 18 | 19 | 20 |
| **(pₘ)** | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, suitably, a minimum of two net positive charges and more preferably a minimum of three net positive charges.

It is also important that the aromatic-cationic peptides have a minimum number of aromatic groups in comparison to the total number of net positive charges (pₜ). The minimum number of aromatic groups will be referred to below as (a). Naturally occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

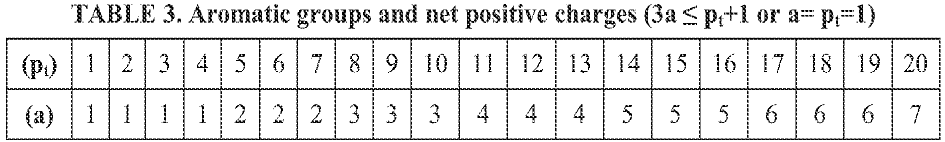

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

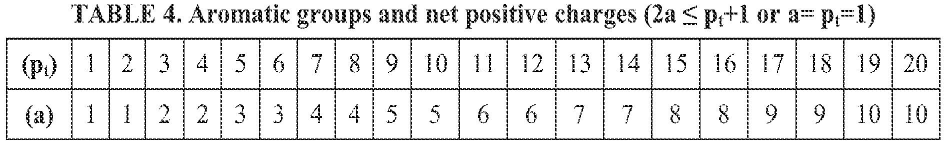

In another embodiment, the number of aromatic groups (a) and the total number of net positive charges (pₜ) are equal.

Carboxyl groups, especially the terminal carboxyl group of a C-terminal amino acid, are suitably amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethylamido, N-methyl-N-ethylamido, N-phenylamido or N-phenyl-N-ethylamido group. The free carboxylate groups of the asparagine, glutamine, aspartic acid, and glutamic acid residues not occurring at the C-terminus of the aromatic-cationic peptides may also be amidated wherever they occur within the peptide. The amidation at these internal positions may be with ammonia or any of the primary or secondary amines described above.

In one embodiment, the aromatic-cationic peptide is a tripeptide having two net positive charges and at least one aromatic amino acid. In a particular embodiment, the aromatic-cationic peptide is a tripeptide having two net positive charges and two aromatic amino acids.

Aromatic-cationic peptides include, but are not limited to, the following peptide examples:
Lys-D-Arg-Tyr-NH₂
Phe-D-Arg-His
D-Tyr-Trp-Lys-NH₂
Trp-D-Lys-Tyr-Arg-NH₂
Tyr-His-D-Gly-Met
Phe-Arg-D-His-Asp
Tyr-D-Arg-Phe-Lys-Glu-NH₂
Met-Tyr-D-Lys-Phe-Arg
D-His-Glu-Lys-Tyr-D-Phe-Arg
Lys-D-Gln-Tyr-Arg-D-Phe-Trp-NH₂
Phe-D-Arg-Lys-Trp-Tyr-D-Arg-His
Gly-D-Phe-Lys-Tyr-His-D-Arg-Tyr-NH₂
Val-D-Lys-His-Tyr-D-Phe-Ser-Tyr-Arg-NH₂
Trp-Lys-Phe-D-Asp-Arg-Tyr-D-His-Lys
Lys-Trp-D-Tyr-Arg-Asn-Phe-Tyr-D-His-NH₂
Thr-Gly-Tyr-Arg-D-His-Phe-Trp-D-His-Lys
Asp-D-Trp-Lys-Tyr-D-His-Phe-Arg-D-Gly-Lys-NH₂
D-His-Lys-Tyr- D-Phe-Glu-D-Asp-D-His-D-Lys-Arg-Trp-NH₂
Ala-D-Phe-D-Arg-Tyr-Lys-D-Trp-His-D-Tyr-Gly-Phe
Tyr-D-His-Phe- D-Arg-Asp-Lys- D-Arg-His-Trp-D-His-Phe
Phe-Phe-D-Tyr-Arg-Glu-Asp-D-Lys-Arg-D-Arg-His-Phe-NH₂
Phe-Try-Lys-D-Arg-Trp-His-D-Lys-D-Lys-Glu-Arg-D-Tyr-Thr
Tyr-Asp-D-Lys-Tyr-Phe- D-Lys- D-Arg-Phe-Pro-D-Tyr-His-Lys
Glu-Arg-D-Lys-Tyr-D-Val-Phe-D-His-Trp-Arg-D-Gly-Tyr-Arg-D-Met-NH₂
Arg-D-Leu-D-Tyr-Phe-Lys-Glu-D-Lys-Arg-D-Trp-Lys-D-Phe-Tyr-D-Arg-Gly

D-Glu-Asp-Lys-D-Arg-D-His-Phe-Phe-D-Val-Tyr-Arg-Tyr-D-Tyr-Arg-His-Phe-
NH2

Asp-Arg-D-Phe-Cys-Phe-D-Arg-D-Lys-Tyr-Arg-D-Tyr-Trp-D-His-Tyr-D-Phe-Lys-
Phe

His-Tyr-D-Arg-Trp-Lys-Phe-D-Asp-Ala-Arg-Cys-D-Tyr-His-Phe-D-Lys-Tyr-His-
Ser-NH2

Gly-Ala-Lys-Phe-D-Lys-Glu-Arg-Tyr-His-D-Arg-D-Arg-Asp-Tyr-Trp-D-His-Trp-
His-D-Lys-Asp

Thr-Tyr-Arg-D-Lys-Trp-Tyr-Glu-Asp-D-Lys-D-Arg-His-Phe-D-Tyr-Gly-Val-Ile-D-
His-Arg-Tyr-Lys-NH2

In one embodiment, the peptides have mu-opioid receptor agonist activity (*i.e*., they activate the mu-opioid receptor). Mu-opioid activity can be assessed by radioligand binding to cloned mu-opioid receptors or by bioassays using the guinea pig ileum (Schiller et al., Eur J Med Chem, 35:895-901, 2000; Zhao et al., J Pharmacol Exp Ther, 307:947-954, 2003). Activation of the mu-opioid receptor typically elicits an analgesic effect. In certain instances, an aromatic-cationic peptide having mu-opioid receptor agonist activity is preferred. For example, during short-term treatment, such as in an acute disease or condition, it may be beneficial to use an aromatic-cationic peptide that activates the mu-opioid receptor. Such acute diseases and conditions are often associated with moderate or severe pain. In these instances, the analgesic effect of the aromatic-cationic peptide may be beneficial in the treatment regimen of the human patient or other mammal. An aromatic-cationic peptide which does not activate the mu-opioid receptor, however, may also be used with or without an analgesic, according to clinical requirements.

Alternatively, in other instances, an aromatic-cationic peptide that does not have mu-opioid receptor agonist activity is preferred. For example, during long-term treatment, such as in a chronic disease state or condition, the use of an aromatic-cationic peptide that activates the mu-opioid receptor may be contraindicated. In these instances, the potentially adverse or addictive effects of the aromatic-cationic peptide may preclude the use of an aromatic-cationic peptide that activates the mu-opioid receptor in the treatment regimen of a human patient or other mammal. Potential adverse effects may include sedation, constipation and respiratory depression. In such instances an aromatic-cationic peptide that does not activate the mu-opioid receptor may be an appropriate treatment.

Peptides which have mu-opioid receptor agonist activity are typically those peptides which have a tyrosine residue or a tyrosine derivative at the N-terminus (*i.e*., the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltryosine (Hmt).

In one embodiment, a peptide that has mu-opioid receptor agonist activity has the formula Tyr-D-Arg-Phe-Lys-NH₂. This peptide has a net positive charge of three, contributed by the amino acids tyrosine, arginine, and lysine and has two aromatic groups contributed by the amino acids phenylalanine and tyrosine. The tyrosine can be a modified derivative of tyrosine such as in 2',6'-dimethyltyrosine to produce the compound having the formula 2',6'-Dmt-D-Arg-Phe-Lys-NH₂. This peptide has a molecular weight of 640 and carries a net three positive charge at physiological pH. The peptide readily penetrates the plasma membrane of several mammalian cell types in an energy-independent manner (Zhao et al., J. Pharmacol Exp Ther., 304:425-432, 2003).

Peptides that do not have mu-opioid receptor agonist activity generally do not have a tyrosine residue or a derivative of tyrosine at the N-terminus (*i*.*e*., amino acid position 1). The amino acid at the N-terminus can be any naturally occurring or non-naturally occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

An example of an aromatic-cationic peptide that does not have mu-opioid receptor agonist activity has the formula Phe-D-Arg-Phe-Lys-NH₂. Alternatively, the N-terminal phenylalanine can be a derivative of phenylalanine such as 2',6'-dimethylphenylalanine (2'6'-Dmp). In one embodiment, a peptide with 2',6'-dimethylphenylalanine at amino acid position 1 has the formula 2',6'-Dmp-D-Arg-Phe-Lys-NH₂. In one embodiment, the amino acid sequence is rearranged such that Dmt is not at the N-terminus. An example of such an aromatic-cationic peptide that does not have mu-opioid receptor agonist activity has the formula D-Arg-2'6'-Dmt-Lys-Phe-NH₂.

The peptides mentioned herein and their derivatives can further include functional analogs. A peptide is considered a functional analog if the analog has the same function as the stated peptide. The analog may, for example, be a substitution variant of a peptide, wherein one or more amino acids are substituted by another amino acid. Suitable substitution variants of the peptides include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) His (H).

Substitutions of an amino acid in a peptide by another amino acid in the same group is referred to as a conservative substitution and may preserve the physicochemical characteristics of the original peptide. In contrast, substitutions of an amino acid in a peptide by another amino acid in a different group is generally more likely to alter the characteristics of the original peptide.

In some embodiments, one or more naturally occurring amino acids in the aromatic-cationic peptides are substituted with amino acid analogs. Examples of analogs that activate mu-opioid receptors include, but are not limited to, the aromatic-cationic peptides shown in Table 5.

**TABLE 5. Peptide Analogs with Mu-Opioid Activity**

| **Amino Acid Position 1** | **Amino Acid Position 2** | **Amino Acid Position 3** | **Amino Acid Position 4** | **C-Terminal Modification** |
|---|---|---|---|---|
| Tvr | D-Arg | Phe | Lys | NH₂ |
| Tyr | D-Arg | Phe | Orn | NH₂ |
| Tyr | D-Arg | Phe | Dab | NH₂ |
| Tyr | D-Arg | Phe | Dap | NH₂ |
| 2'6'Dmt | D-Arp | Phe | Lys | NH₂ |
| 2'6'Dmt | D-Arg | Phe | Lys-NH(CH₂)₂-NH-dns | NH₂ |
| 2'6'Dmt | D-Arg | Phe | Lys-NH(CH₂)₂-NH-atn | NH₂ |
| 2'6'Dmt | D-Arg | Phe | dnsLys | NH₂ |
| 2'6'Dmt | D-Cit | Phe | Lys | NH₂ |
| 2'6'Dmt | D-Cit | Phe | Ahp | NH₂ |
| 2'6'Dmt | D-Arg | Phe | Orn | NH₂ |
| 2'6'Dmt | D-Arg | Phe | Dab | NH₂ |
| 2'6'Dmt | D-Arg | Phe | Dap | NH₂ |
| 2'6'Dmt | D-Arg | Phe | Ahp(2-aminoheptanoic acid) | NH₂ |
| Bio-2'6'Dmt | D-Arp | Phe | Lys | NH₂ |
| 3'5'Dmt | D-Arg | Phe | Lys | NH₂ |
| 3'5'Dmt | D-Arg | Phe | Orn | NH₂ |
| 3'5'Dmt | D-Arg | Phe | Dab | NH₂ |
| 3'5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2'6'Dmt | D-Arg | Tyr | Lys | NH₂ |
| 2'6'Dmt | D-Arg | Tyr | Orn | NH₂ |
| 2'6Dmt | D-Arg | Tyr | Dab | NH₂ |
| 2'6Dmt | D-Arg | Tyr | Dap | NH₂ |
| 2'6'Dmt | D-Arg | 2'6'Dmt | Lys | NH₂ |
| 2'6Dmt | D-Arg | 2'6'Dmt | Orn | NH₂ |
| 2'6'Dmt | D-Arg | 2'6'Dmt | Dab | NH₂ |
| 2'6'Dmt | D-Arg | 2'6'Dmt | Dap | NH₂ |
| 3'5'Dmt | D-Arg | 3'5'Dmt | Arg | NH₂ |
| 3'5'Dmt | D-Arg | 3'5'Dmt | Lys | NH₂ |
| 3'5'Dmt | D-Arg | 3'5'Dmt | Orn | NH₂ |
| 3'5'Dmt | D-Arg | 3'5'Dmt | Dab | NH₂ |
| Tyr | D-Lys | Phe | Dap | NH₂ |
| Tyr | D-Lys | Phe | Arg | NH₂ |
| Tyr | D-Lys | Phe | Lys | NH₂ |
| Tyr | D-Lys | Phe | Orn | NH₂ |
| 2'6'Dmt | D-Lys | Phe | Dab | NH₂ |
| 2'6'Dmt | D-Lys | Phe | Dap | NH₂ |
| 2'6'Dmt | D-Lys | Phe | Arg | NH₂ |
| 2'6'Dmt | D-Lys | Phe | Lys | NH₂ |
| 3'5'Dmt | D-Lys | Phe | Orn | NH₂ |
| 3'5'Dmt | D-Lys | Phe | Dab | NH₂ |
| 3'5'Dmt | D-Lys | Phe | Dap | NH₂ |
| 3'5'Dmt | D-Lys | Phe | Arg | NH₂ |
| Tyr | D-Lvs | Tyr | Lys | NH₂ |
| Tyr | D-Lys | Tyr | Orn | NH₂ |
| Tyr | D-Lys | Tyr | Dab | NH₂ |
| Tyr | D-Lys | Tyr | Dap | NH₂ |
| 2'6'Dmt | D-Lys | Tyr | Lys | NH₂ |
| 2'6Dmt | D-Lys | Tyr | Orn | NH₂ |
| 2'6Dmt | D-Lys | Tyr | Dab | NH₂ |
| 2'6'Dmt | D-Lys | Tyr | Dap | NH₂ |
| 2'6'Dmt | D-Lys | 2'6'Dmt | Lys | NH₂ |
| 2'6'Dmt | D-Lys | 2'6'Dmt | Orn | NH₂ |
| 2'6'Dmt | D-Lys | 2'6'Dmt | Dab | NH₂ |
| 2'6'Dmt | D-Lys | 2'6'Dmt | Dap | NH₂ |
| 2'6'Dmt | D-Arg | Phe | dnsDap | NH₂ |
| 2'6Dmt | D-Arg | Phe | atnDap | NH₂ |
| 3'5'Dmt | D-Lys | 3'5'Dmt | Lys | NH₂ |
| 3'5'Dmt | D-Lys | 3'5'Dmt | Orn | NH₂ |
| 3'5'Dmt | D-Lys | 3'5'Dmt | Dab | NH₂ |
| 3'5'Dmt | D-Lys | 3'5'Dmt | Dap | NH₂ |
| Tyr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2'6Dmt | D-Arg | Phe | Arg | NH₂ |
| 2'6Dmt | D-Lys | Phe | Arg | NH₂ |
| 2'6'Dmt | D-Orn | Phe | Arg | NH₂ |
| 2'6Dmt | D-Dab | Phe | Arg | NH₂ |
| 3'5'Dmt | D-Dap | Phe | Arg | NH₂ |
| 3'5'Dmt | D-Arg | Phe | Arg | NH₂ |
| 3'5'Dmt | D-Lys | Phe | Arg | NH₂ |
| 3'5'Dmt | D-Orn | Phe | Arg | NH₂ |
| Tyr | D-Lys | Tyr | Arg | NH₂ |
| Tyr | D-Orn | Tyr | Arg | NH₂ |
| Tyr | D-Dab | Tyr | Arg | NH₂ |
| Tyr | D-Dap | Tyr | Arg | NH₂ |
| 2'6'Dmt | D-Arg | 2'6'Dmt | Arg | NH₂ |
| 2'6Dmt | D-Lys | 2'6'Dmt | Arg | NH₂ |
| 2'6'Dmt | D-Orn | 2'6'Dmt | Arg | NH₂ |
| 2'6'Dmt | D-Dab | 2'6'Dmt | Arg | NH₂ |
| 3'5'Dmt | D-Dap | 3'5'Dmt | Arg | NH₂ |
| 3'5'Dmt | D-Arg | 3'5'Dmt | Arg | NH₂ |
| 3'5'Dmt | D-Lys | 3'5'Dmt | Arg | NH₂ |
| 3'5'Dmt | D-Orn | 3'5'Dmt | Arg | NH₂ |
| Mmt | D-Arg | Phe | Lys | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-Arg | Phe | Orn | NH₂ |
| Tmt | D-Arg | Phe | Dab | NH₂ |
| Tmt | D-Arg | Phe | Dap | NH₂ |
| Hmt | D-Arg | Phe | Lys | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Lys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lvs | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |

| | | | | |
|---|---|---|---|---|
| Dab = diaminobutyric Dap = diaminopropionic acid Dmt = dimethyltyrosine Mmt = 2'-methyltyrosine Tmt = N, 2',6'-trimethyltyrosine Hmt = 2'-hydroxy,6'-methyltyrosine dnsDap = β-dansyl-L-α,β-diaminopropionic acid atnDap = β-anthraniloyl-L-α,β-diaminopropionic acid Bio = biotin | | | | |

Examples of analogs that do not activate mu-opioid receptors include, but are not limited to, the aromatic-cationic peptides shown in Table 6.

**TABLE 6. Peptide Analogs Lacking Mu-Opioid Activity**

| **Amino Acid Position 1** | **Amino Add Position 2** | **Amino Acid Position 3** | **Amino Acid Position 4** | **C-Terminal Modification** |
|---|---|---|---|---|
| D-Arg | Dmt | Lys | Phe | NH₂ |
| D-Arg | Dmt | Phe | Lys | NH₂ |
| D-Arg | Phe | Lys | Dmt | NH₂ |
| D-Arg | Phe | Dmt | Lys | NH₂ |
| D-Arg | Lys | Dmt | Phe | NH₂ |
| D-Arg | Lys | Phe | Dmt | NH₂ |
| Phe | Lys | Dmt | D-Arg | NH₂ |
| Phe | Lys | D-Arg | Dmt | NH₂ |
| Phe | D-Arg | Phe | Lys | NH₂ |
| Phe | D-Arg | Dmt | Lys | NH₂ |
| Phe | D-Arg | Lys | Dmt | NH₂ |
| Phe | Dmt | D-Arg | Lys | NH₂ |
| Phe | Dmt | Lys | D-Arg | NH₂ |
| Lys | Phe | D-Arg | Dmt | NH₂ |
| Lys | Phe | Dmt | D-Arg | NH₂ |
| Lys | Dmt | D-Arg | Phe | NH₂ |
| Lys | Dmt | Phe | D-Arg | NH₂ |
| Lys | D-Arg | Phe | Dmt | NH₂ |
| Lys | D-Arg | Dmt | Phe | NH₂ |
| D-Arg | Dmt | D-Arg | Phe | NH₂ |
| D-Arg | Dmt | D-Arg | Dmt | NH₂ |
| D-Arg | Dmt | D-Arg | Tyr | NH₂ |
| D-Arg | Dmt | D-Arg | Trp | NH₂ |
| Trp | D-Arg | Phe | Lys | NH₂ |
| Trp | D-Arg | Tyr | Lys | NH₂ |
| Trp | D-Arg | Trp | Lys | NH₂ |
| Trp | D-Arg | Dmt | Lys | NH₂ |
| D-Arg | Trp | Lys | Phe | NH₂ |
| D-Arg | Trp | Phe | Lys | NH₂ |
| D-Arg | Trp | Lys | Dmt | NH₂ |
| D-Arg | Trp | Dmt | Lys | NH₂ |
| D-Arg | Lys | Trp | Phe | NH₂ |
| D-Arg | Lys | Trp | Dmt | NH₂ |
| Cha | D-Arg | Phe | Lys | NH₂ |
| Ala | D-Arg | Phe | Lys | NH₂ |

| | | | | |
|---|---|---|---|---|
| Cha = cyclohexyl alanine | | | | |

The amino acids of the peptides shown in Table 5 and 6 may be in either the L- or the D- configuration.

The peptides may be synthesized by any of the methods well known in the art. Suitable methods for chemically synthesizing the protein include, for example, those described by Stuart and Young in Solid Phase Peptide Synthesis, Second Edition, Pierce Chemical Company (1984), and in Methods Enzymol., 289, Academic Press, Inc, New York (1997).

### Prophylactic and Therapeutic Uses of Aromatic-Cationic Peptides.

*General*. The aromatic-cationic peptides described herein are useful to prevent or treat disease. Specifically, the disclosure provides for both prophylactic and therapeutic methods of treating a subject at risk of (or susceptible to) vessel occlusion injury or cardiac ischemia-reperfusion injury. Accordingly, the present methods provide for the prevention and/or treatment of vessel occlusion injury or cardiac ischemia-reperfusion injury in a subject by administering an effective amount of an aromatic-cationic peptide to a subject in need thereof, and performing a CABG procedure.

*Determination of the Biological Effect of the Aromatic-Cationic Peptide-Based Therapeutic*. In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative animal models, to determine if a given aromatic-cationic peptide-based therapeutic exerts the desired effect in preventing or treating ischemia-reperfusion injury. Compounds for use in therapy can be tested in suitable animal model systems including, but not limited to rats, mice, chicken, pigs, cows, monkeys, rabbits, and the like, prior to testing in human subjects. Similarly, for *in vivo* testing, any of the animal model systems known in the art can be used prior to administration to human subjects.

*Prophylactic Methods*. In one aspect, the invention provides a method for preventing, in a subject, vessel occlusion injury by administering to the subject an aromatic-cationic peptide that prevents the initiation or progression of the condition. Subjects at risk for vessel occlusion injury can be identified by, *e.g*., any or a combination of diagnostic or prognostic assays as described herein. In prophylactic applications, pharmaceutical compositions or medicaments of aromatic-cationic peptides are administered to a subject susceptible to, or otherwise at risk of a disease or condition in an amount sufficient to eliminate or reduce the risk, lessen the severity, or delay the outset of the disease, including biochemical, histologic and/or behavioral symptoms of the disease, its complications and intermediate pathological phenotypes presenting during development of the disease. Administration of a prophylactic aromatic-cationic can occur prior to the manifestation of symptoms characteristic of the aberrancy, such that a disease or disorder is prevented or, alternatively, delayed in its progression. The appropriate compound can be determined based on screening assays described above. In some embodiments, the peptides are administered in sufficient amounts to prevent renal or celebral complications from CABG.

*Therapeutic Methods*. Another aspect of the technology includes methods of treating vessel occlusion injury in a subject for therapeutic purposes. In therapeutic applications, compositions or medicaments are administered to a subject suspected of, or already suffering from such a disease in an amount sufficient to cure, or at least partially arrest, the symptoms of the disease, including its complications and intermediate pathological phenotypes in development of the disease. As such, the invention provides methods of treating an individual afflicted with cardiac ischemia-reperfusion injury by administering an effective amount of an aromatic-cationic peptide and performing a CABG procedure.

### Modes of Administration and Effective Dosages

Any method known to those in the art for contacting a cell, organ or tissue with a peptide may be employed. The effective amount may be determined during pre-clinical trials and clinical trials by methods familiar to physicians and clinicians. An effective amount of a peptide useful in the methods may be administered to a mammal in need thereof by any of a number of well-known methods for administering pharmaceutical compounds. The peptide may be administered systemically or locally.

The peptide may be formulated as a pharmaceutically acceptable salt. The term "pharmaceutically acceptable salt" means a salt prepared from a base or an acid which is acceptable for administration to a patient, such as a mammal (*e.g*., salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (e.g., citric, gluconic, glycolic, lactic, lactobionic, malic, and tartaric acids), aliphatic monocarboxylic acids (*e.g*., acetic, butyric, formic, propionic and trifluoroacetic acids), amino acids (*e.g*., aspartic and glutamic acids), aromatic carboxylic acids (*e.g*., benzoic, p-chlorobenzoic, diphenylacetic, gentisic, hippuric, and triphenylacetic acids), aromatic hydroxyl acids (*e.g*., o-hydroxybenzoic, p-hydroxybenzoic, 1-hydroxynaphthalene-2-carboxylic and 3-hydroxynaphthalene-2-carboxylic acids), ascorbic, dicarboxylic acids (*e.g*., fumaric, maleic, oxalic and succinic acids), glucoronic, mandelic, mucic, nicotinic, orotic, pamoic, pantothenic, sulfonic acids (*e.g*., benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, and the like.

The aromatic-cationic peptides described herein can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with its intended route of administration. Examples of routes of administration include parenteral (e.g., intravenous, intradermal, intraperitoneal or subcutaneous), oral, inhalation, transdermal (topical), intraocular, iontophoretic, and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic. For convenience of the patient or treating physician, the dosing formulation can be provided in a kit containing all necessary equipment (*e.g*., vials of drug, vials of diluent, syringes and needles) for a treatment course (*e.g*., 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

The aromatic-cationic peptide compositions can include a carrier, which can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e*.*g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser which contains a suitable propellant, *e.g*., a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed my iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. As one skilled in the art would appreciate, there are a variety of methods to prepare liposomes. (*See* Lichtenberg et al., Methods Biochem. Anal., 33:337-462 (1988); Anselem et al., Liposome Technology, CRC Press (1993)). Liposomal formulations can delay clearance and increase cellular uptake (*See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g*., a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g*., collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. (*See* Reddy, Ann. Pharmacother., 34(7-8):915-923 (2000)). A polymer formulation for human growth hormone (hGH) has been used in clinical trials. (*See* Kozarich and Rich, Chemical Biology, 2:548-552 (1998)).

Examples of polymer microsphere sustained release formulations are described in PCT publication WO 99/15154 (Tracy et al.), U.S. Pat. Nos. 5,674,534 and 5,716,644 (both to Zale et al.), PCT publication WO 96/40073 (Zale et al.), and PCT publication WO 00/38651 (Shah et al.). U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylacetic acid. Such formulations can be prepared using known techniques. The materials can also be obtained commercially, e.g., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g*., Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi et al., Cancer Lett., 100:63-69 (1996), describes the use of fusogenic liposomes to deliver a protein to cells both *in vivo* and *in vitro*.

Dosage, toxicity and therapeutic efficacy of the therapeutic agents can be determined by standard pharmaceutical procedures in cell cultures or experimental animals, *e.g*., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 (*i.e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.1-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, and preferably until the subject shows partial or complete amelioration of symptoms of disease. Thereafter, the patient can be administered a prophylactic regime.

In an exemplary embodiment, the subject is administered the peptide by intravenous infusion at about 0.001 to about 1 mg/kg/hr, *i*.*e*., about 0.005, about 0.01, about 0.025, about 0.05, about 0.10, about 0.25, or about 0.5 mg/kg/hour. The intravenous infusion maybe started prior to or after reperfusion of the tissue.

In some embodiments, a therapeutically effective amount of an aromatic-cationic peptide may be defined as a concentration of peptide at the target tissue of 10⁻¹² to 10⁻⁶ molar, e.g., approximately 10⁻⁷ molar. This concentration may be delivered by systemic doses of 0.001 to 100 mg/kg or equivalent dose by body surface area. The schedule of doses would be optimized to maintain the therapeutic concentration at the target tissue, most preferably by single daily or weekly administration, but also including continuous administration (*e.g*., parenteral infusion or transdermal application).

In some embodiments, the dosage of the aromatic-cationic peptide is provided at a "low," "mid," or "high" dose level. In one embodiment, the low dose is provided from about 0.001 to about 0.5 mg/kg/h, suitably from about 0.01 to about 0.1 mg/kg/h. In one embodiment, the mid-dose is provided from about 0.1 to about 1.0 mg/kg/h, suitably from about 0.1 to about 0.5 mg/kg/h. In one embodiment, the high dose is provided from about 0.5 to about 10 mg/kg/h, suitably from about 0.5 to about 2 mg/kg/h. The intravenous infusion may be started prior to or after reperfusion of the tissue. In some embodiments, the subject may receive in IV bolus injection prior to reperfusion of the tissue. In one embodiment, the peptide is administered in conjunction with a cardioplegia solution. In one embodiment, the peptide is administered as part of the priming solution in a heart lung machine during cardiopulmonary bypass.

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance present methods can be any mammal, including, for example, farm animals, such as sheep, pigs, cows, and horses; pet animals, such as dogs and cats; laboratory animals, such as rats, mice and rabbits. In suitable embodiment, the mammal is a human.

### EXAMPLES

The present invention is further illustrated by the following example, which should not be construed as limiting in any way.

### Example 1. Effects of Aromatic-Cationic Peptides in Protecting Against Vessel Occlusion Injury in a Rabbit Model.

The effects of aromatic-cationic peptides in protecting against a vessel occlusion injury in a rabbit model were investigated. The myocardial protective effect of the peptide D-Arg-2'6'-Dmt-Lys-Phe-NH₂ were demonstrated by this Example.

### Experimental Methods

New Zealand white rabbits were used in this study. The rabbits were males and > 10 weeks in age. Environmental controls in the animal rooms were set to maintain temperatures of 61° to 72°F and relative humidity between 30% and 70%. Room temperature and humidity were recorded hourly, and monitored daily. There were approximately 10 - 15 air exchanges per hour in the animal rooms. Photoperiod was 12-hr light/12-hr dark (*via* fluorescent lighting) with exceptions as necessary to accommodate dosing and data collection. Routine daily observations were performed. Harlan Teklad, Certified Diet (2030C), rabbit diet was provided approximately 180 grams per day from arrival to the facility. In addition, fresh fruits and vegetables were given to the rabbit 3 times a week.

The peptide D-Arg-2'6'-Dmt-Lys-Phe-NH₂ (sterile lyophilized powder) was used as the test article. Dosing solutions were formulated at no more than 1 mg/ml, and were delivered *via* continuous infusion (IV) at a constant rate (*e.g*., 50 µL/kg/min). Normal saline (0.9% NaCl) was used as a control.

The test/vehicle articles were given intravenously, under general anesthesia, in order to mimic the expected route of administration in the clinical setting of AMI and PTCA. Intravenous infusion was administered *via* a peripheral vein using a Kd Scientific infusion pump (Holliston, MA 01746) at a constant volume (*e.g*., 50 µL/kg/min).

The study followed a predetermined placebo and sham controlled design. In short, 10-20 healthy, acclimatized, male rabbits were assigned to one of three study arms (approximately 2-10 animals per group). Arm A (n = 10, CTRL/PLAC) includes animals treated with vehicle (vehicle; VEH, IV); Arm B (n = 10, treated) includes animals treated with peptide; Arm C (n = 2, SHAM) includes sham-operated time-controls treated with vehicle (vehicle; VEH, IV) or peptide.

**Table 7. Study Design.**

| **Group** | **Study Group** | **Ischemia Time** | **Reperfusion Time** |
|---|---|---|---|
| A | CONTROL/PLACEBO | 30 Min (Last 20 Min. With Placebo) | 180 Min of Placebo |
| B | PEPTIDE | 30 Min (Last 20 Min. With Peptide) | 180 Min of Peptide |
| C | SHAM (FOR SURGERY WITHOUT ISCHEMIA) | 0 Min (Last 20 Min. With Placebo) | 180 Min of Placebo (Vehicle) or Peptide |

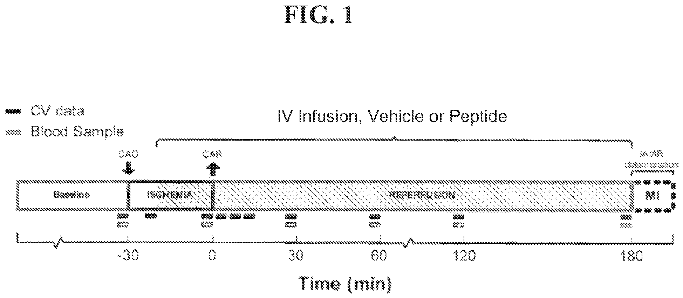

In all cases, treatments were started approximately 10 min after the onset of a 30 min ischemic insult (coronary occlusion) and continued for up to 3 h following reperfusion. In all cases, cardiovascular function was monitored both prior to and during ischemia, as well as for up to 180 min (3 h) post-reperfusion. The experiments were terminated 3 h post-reperfusion (end of study); irreversible myocardial injury (infarct size by histomorphometery) at this time-point was evaluated, and was the primary-end-point of the study. The study design is summarized in Table 7 and FIG. 1.

*Anesthesia*/*Surgical Preparation*. General anesthesia was induced intramuscularly (IM) with a ketamine (∼35 -50 mg/kg)/xylazine (∼5 -10 mg/kg) mixture. A venous catheter was placed in a peripheral vein (*e.g*., ear) for the administration of anesthetics. In order to preserve autonomic function, anesthesia was maintained with continuous infusions of propofol (∼8 - 30 mg/kg/hour) and ketamine (∼1.2 - 2.4 mg/kg/hr). A cuffed tracheal tube was placed *via* a tracheotomy (ventral midline incision) and used to mechanically ventilate the lungs with a 95% O₂/5% CO₂ mixture *via* a volume-cycled animal ventilator (∼40 breaths/minute with a tidal volume of ∼12.5 ml/kg) in order to sustain PaCO₂ values broadly within the physiological range.

Once a surgical plane of anesthesia was reached, either transthoracic or needle electrodes forming two standard ECG leads (*e.g*., lead II, aVF, V2) were placed. A cervical cut-down exposed a carotid artery, which was isolated, dissected free from the surrounding tissue and cannulated with a dual-sensor high-fidelity micromanometer catheter (Millar Instruments); the tip of this catheter was advanced into the left-ventricle (LV) retrogradely across the aortic valve, in order to simultaneously determine aortic (root, proximal transducer) and left-ventricular (distal transducer) pressures. The carotid cut-down also exposed the jugular vein, which was cannulated with a hollow injection catheter (for blood sampling). Finally, an additional venous catheter was placed in a peripheral vein (*e*.*g*., ear) for the administration of vehicle/test articles.

Subsequently, the animals were placed in right-lateral recumbence and the heart was exposed *via* a midline thoracotomy and a pericardiotomy. The heart was suspended on a pericardial cradle in order to expose the left circumflex (LCX) and the left-anterior descending (LAD) coronary arteries. Silk ligatures were loosely placed (using a taper-point needle) around the proximal LAD and if necessary, depending on each animal's coronary anatomy, around one or more branches of the LCX marginal coronary arteries. Tightening of these snares (*via* small pieces of polyethylene tubing) allowed rendering a portion of the left ventricular myocardium temporarily ischemic.

Once instrumentation was completed, hemodynamic stability and proper anesthesia depth were verified/ensured for at least 30 min. Subsequently, the animals were paralyzed with atracurium (∼0.1 to 0.2 mg/kg/hr IV) in order to facilitate hemodynamic/respiratory stability. Following atracurium administration, signs of autonomic hyperactivity and/or changes in BIS values were used to evaluate anesthesia depth and/or to up-titrate the intravenous anesthetics.

*Experimental Protocol*/*Cardiovascular Data Collection*. Immediately following surgical preparation, the animals were heparinized (100 units heparin/kg/h, IV bolus), and after hemodynamic stabilization (for approximately 30 min), baseline data were collected including venous blood for the evaluation of cardiac enzymes/biomarkers as well as of test-article concentrations.

Following hemodynamic stabilization and baseline measurements, the animals were subjected to an acute 30 min ischemic insult by tightening of the LAD/LCX coronary artery snares. Myocardial ischemia was visually confirmed by color (*i.e*., cyanotic) changes in distal distributions of the LAD/LCX and by the onset of electrocardiographic changes. Approximately after 10 min of ischemia, the animals received a continuous infusion of either vehicle (saline) or peptide; ischemia was continued for a additional 20 min (*i.e*., 30 min total) after the start of treatment. Subsequently (*i.e*., after 30 min of ischemia of which the last 20 min overlap with the treatment), the coronary snares were released and the previously ischemic myocardium was reperfused for up to 3 h. Treatment with either vehicle or peptide was continued throughout the reperfusion period. It should be noted that in sham-operated animals the vessel snares were manipulated at the time of ischemia/reperfusion onset, but were not either tightened or loosened.

Cardiovascular data collection occurred at 11 pre-determined time-points: post-instrumentation/stabilization (*i.e*., baseline), after 10 and 30 min of ischemia, as well as at 5, 15, 30, 60, 120, and 180 min post-reperfusion. Throughout the experiments, analog signals were digitally sampled (1000 Hz) and recorded continuously with a data acquisition system (IOX; EMKA Technologies), and the following parameters were determined at the above-mentioned time-points: (1) from bipolar transthoracic ECG (*e.g*., Lead II, aVF): rhythm (arrhythmia quantification/classification), RR, PQ, QRS, QT, QTc, short-term QT instability, and QT:TQ (restitution); (2) from solid-state manometer in aorta (Millar): arterial/aortic pressure (AoP); and (3) from solid-state manometer in the LV (Millar): left-ventricular pressures (ESP, EDP) and derived indices (dP/dtmax, dP/dtmin, Vmax, and tau). In addition, in order to determine/quantify the degree of irreversible myocardial injury (*i.e*., infarction) resulting from the I/R insult with and without peptide treatment, cardiac biomarkers as well as infarct area were evaluated.

*Blood Samples*. Venous (<3 mL) whole blood samples were collected for both pharmaco-kinetic (PK) analysis as well as for the evaluation of myocardial injury *via* cardiac biomarker analyses at six data-collection time-points: baseline, 30 min of ischemia, as well as 30, 60, 120 and 180 min post-reperfusion. In addition, three arterial (∼0.5 mL) whole blood samples were collected at baseline, 60 min of ischemia, as well as the 60 and 180 min post-reperfusion for the determination of blood-gases, the arterial samples were collected into blood gas syringes and used for the measurement of blood-gases *via* an I-Stat analyzer/cartridges (CG4+).

*Histopathology*/*Histomorphometery.* At the completion of the protocol, irreversible myocardial injury (*i.e*., infarction) resulting from the I/R insult was evaluated. In short, the coronary snares were retightened and Evan's blue dye (1 mL/kg; Sigma, St. Louis, MO) was injected intravenously to delineate the myocardial area-at-risk (AR) during ischemia. Approximately 5 min later, the heart was arrested (by an injection of potassium chloride into the left atrium), and freshly excised. The LV was sectioned perpendicular to its long axis (from apex to base) into 3 mm thick slices. Subsequently, the slices were incubated for 20 min in 2% triphenyl-tetrazolium-chloride (TTC) at 37°C and fixed in a 10% non-buffered formalin solution (NBF).

Following fixation, the infarct and at-risks areas were delineated/measured digitally. For such purpose, the thickness of each slice was measured with a digital micrometer and later photographed/scanned. All photographs were imported into an image analysis program (Image J; National Institutes of Health), and computer-assisted planometry was performed to determine the overall size of the infarct (I) and at-risk (AR) areas. For each slide, the AR (*i.e*., not stained blue) was expressed as a percentage of the LV area, and the infarct size (I, not stained tissue) was expressed as a percentage of the AR (I/AR). In all cases, quantitative histomorphometery was performed by personnel blinded to the treatment assignment/study-design.

*Animal Observations.* Data were acquired on the EMKA's IOX system using ECG Auto software for analysis (EMKA Technologies). Measurements for all physiological parameters were made manually or automatically from (digital) oscillograph tracings. The mean value from 60 s of data from each targeted time point was used (if possible); however, as mentioned above, signals/tracing was recorded continuously throughout the experiments, in order to allow (if needed) more fine/detailed temporal data analysis (*via* amendments). Additional calculations were performed using Microsoft Excel. Data is presented as means with standard errors.

### Results

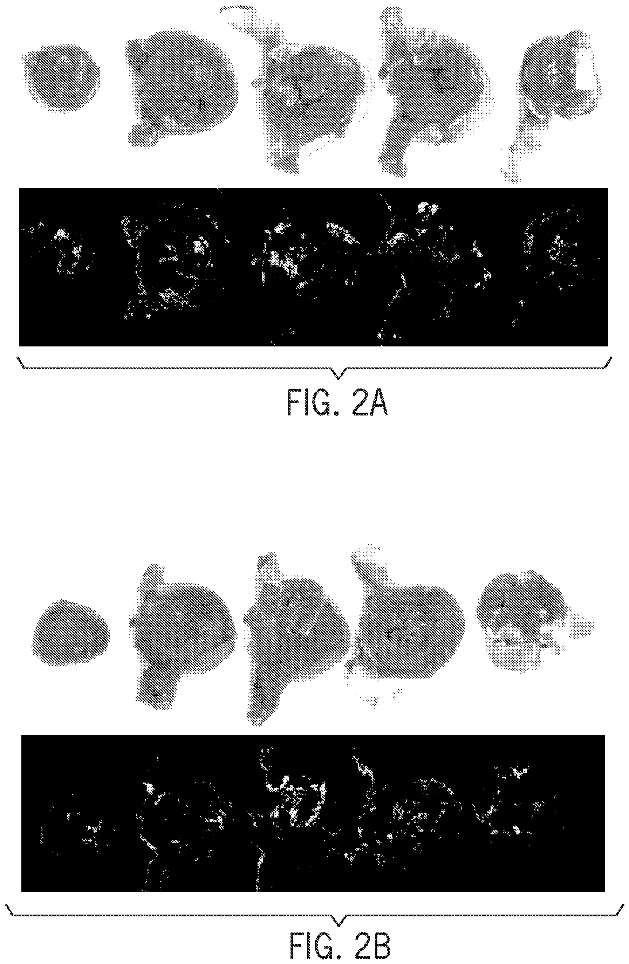

Infarct size from hearts exposed to 30 min ischemia and 3 h reperfusion is shown in FIGs. 2-6. FIGs. 2A and 2B present data showing infarct size for rabbits with a sham for surgery (ligature applied, but not tightened), with placebo or with peptide. The LV was sectioned perpendicular to its long axis (from apex to base) into 3 mm thick slices. FIG. 2A is a photograph of heart slices and a computer-generated image highlighting infarct size of a sham rabbit treated with a placebo. FIG. 2B is a photograph of heart slices and a computer-generated image highlighting infarct size of a sham rabbit treated with peptide.

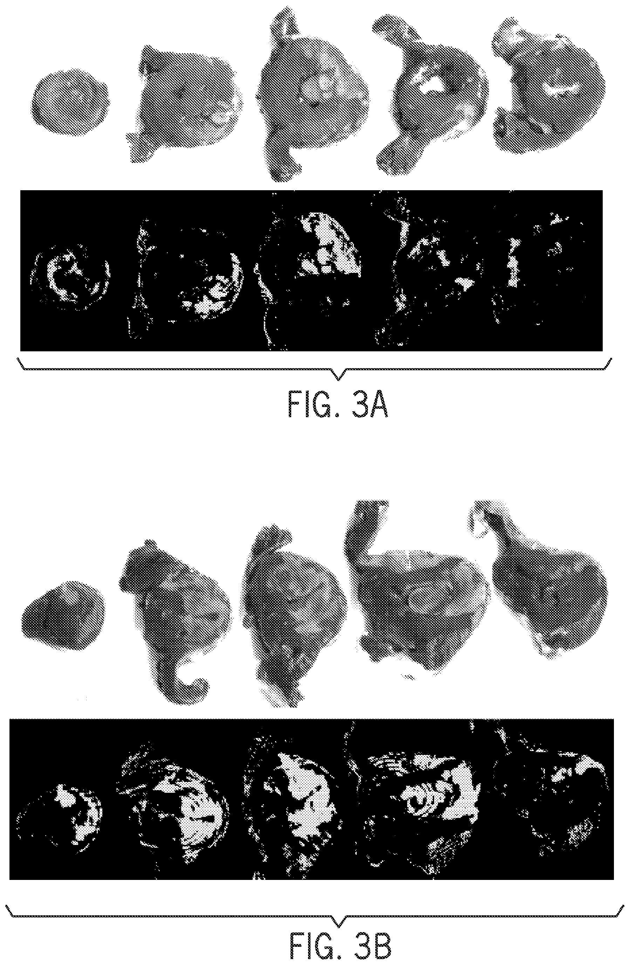

FIGs. 3A and 3B present data showing infarct size for two different control rabbits with induced cardiac ischemia and treated with a placebo. Each figure shows a photograph of heart slices and a computer-generated image highlighting infarct size.

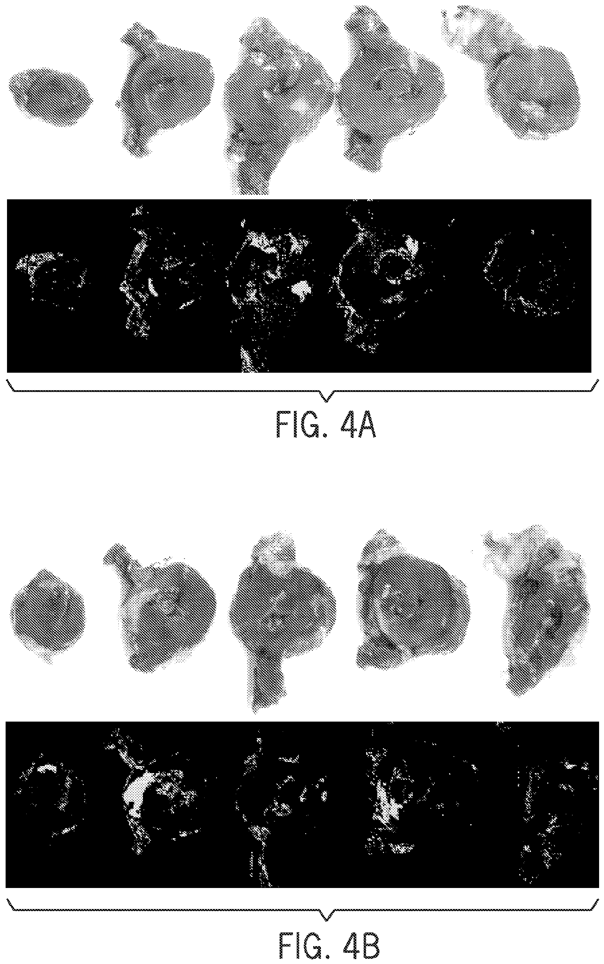

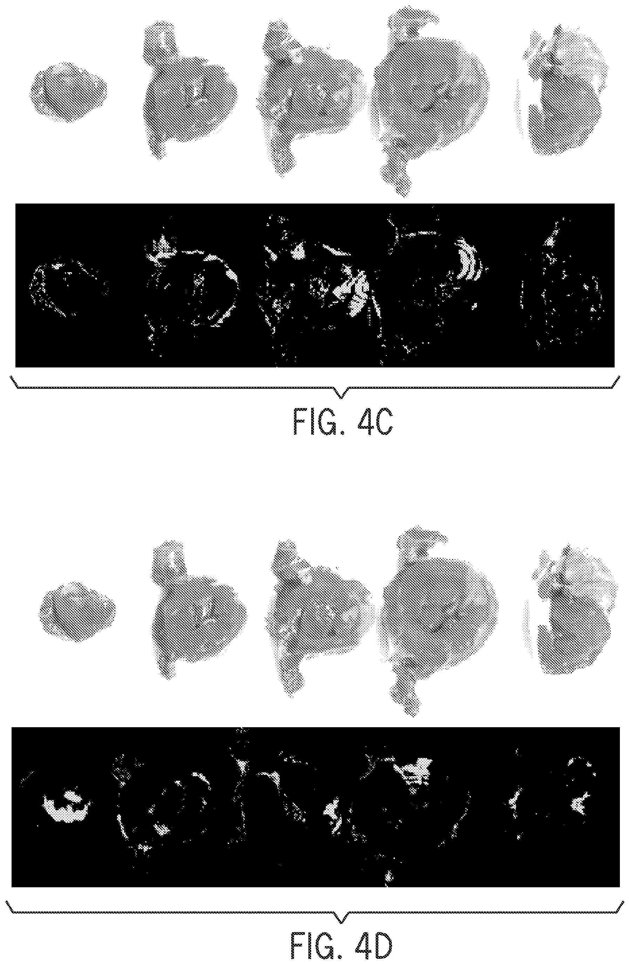

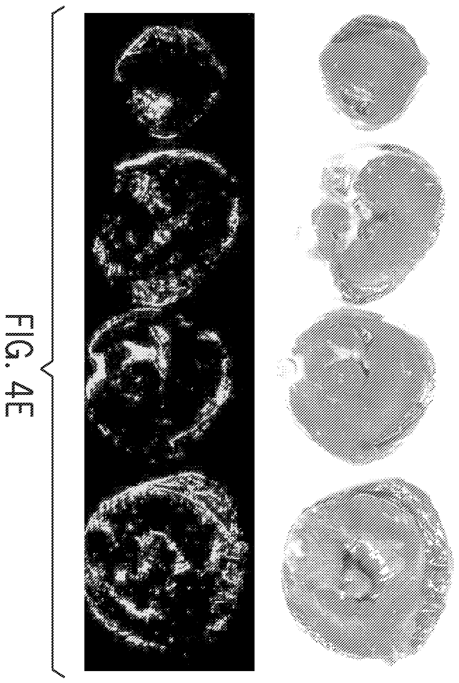

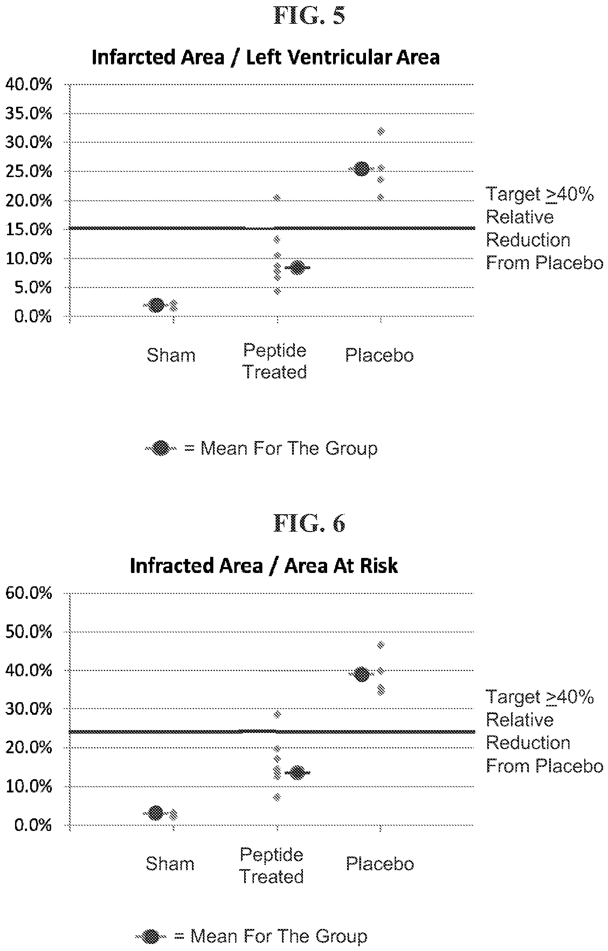

FIGs. 4A, 4B, 4C, 4D, and 4E present data showing infarct size for five different rabbits with induced cardiac ischemia and treated with the peptide. Administration of peptide resulted in decreased infarct size compared to the control. Table 8 presents data showing the ratios of area of risk to left ventricular area infracted area to left ventricular area, and infracted area to area of risk for each of the animals used in this study. FIGs. 5-6 present further data showing the ratios of area of risk to left ventricular area infracted area to left ventricular area, and infracted area to area of risk in peptide-treated and control subjects.

**Table 8. Histopathology Results of Study Animals**

| **Group** | **Myocardial Area (%)** | | |
|---|---|---|---|
| | **AR/LV** | **IA/LV** | **IA/AR** |
| SHAM (n=2) | 56.0 ± 0.4 | 1.7 ± 0.2 | 2.8 ± 0.3 |
| Peptide (n=10) | 58.2 ± 1.9 | 16.2 ± 3.4 | 24.7 ± 4.7 |
| *% versus placebo* | 6 ± 3 | -24 ± 16 | -32 ± 13 |
| Placebo (n=10) | 55.1 ± 2.4 | 21.5 ± 1.7 | 36.1 ± 1.9 |
| ***p value*** (Peptide vs. Placebo) | | p<0.05 | <0.05 |

These results show that in a standardized rabbit model of acute myocardial ischemia and reperfusion, peptide when administered as an IV continuous infusion beginning at 10 min into a 30 min ischemia period followed by IV continuous infusion for 180 min after reperfusion was able to reduce myocardial infarct size compared to the control group. In the rabbits in which there was a definable response to treatment, the size of the myocardial infarct area was reduced relative to the infarct size noted in control animals. Treatment for less than 3 hours after reperfusion, *i.e.*, 30 min, provided comparable myocardial salvage (data not shown). These results indicate that peptide treatment prevents the occurrence of symptoms of acute cardiac ischemia-reperfusion injury. As such, aromatic-cationic peptides are useful in methods at preventing and treating a vessel occlusion injury in mammalian subjects.

### Example 2. Effects of Peptides in Protecting Against Vessel Occlusion Injury in Humans

This Example will determine whether the administration of D-Arg-2'6'-Dmt-Lys-Phe-NH₂ at the time of revascularization would limit the size of the infarct during acute myocardial infarction.

*Study group.* Men and women, 18 years of age or older, who present after the onset of chest pain, and for whom the clinical decision is made to treat with a revascularization procedure (*e*.*g*., PCI or thrombolytics) are eligible for enrollment. The patient may be STEMI or Non-STEMI. A STEMI patient will present with symptoms suggestive of a cutting off of the blood supply to the myocardium and also if the patient's ECG shows the typical heart attack pattern of ST elevation. The diagnosis is made therefore purely on the basis of symptoms, clinical examination and ECG changes. In the case of a Non-ST elevation heart attack, the symptoms of chest pain can be identical to that of a STEMI, but the important difference is that the patient's ECG does not show the typical ST elevation changes traditionally associated with a heart attack. The patient often has a history of having experienced angina, but the ECG at the time of the suspected attack may show no abnormality at all. The diagnosis is suspected on the history and symptoms and is confirmed by a blood test which shows a rise in the concentration of substances called cardiac enzymes in the blood.

*Angiography and Revascularization.* Left ventricular and coronary angiography is performed with the use of standard techniques, just before revascularization. Revascularization is performed by PCI with the use of direct stenting. Alternative revascularization procedures include, but are not limited to, balloon angioplasty; percutaneous transluminal coronary angioplasty; and directional coronary atherectomy

*Experimental Protocol.* After coronary angiography is performed but before the stent is implanted, patients who meet the enrollment criteria are randomly assigned to either the control group or the peptide group. Randomization is performed with the use of a computer-generated randomization sequence. Less than 10 min before direct stenting, the patients in the peptide group receive an intravenous bolus injection of D-Arg-2'6'-Dmt-Lys-Phe-NH₂. The peptide is dissolved in normal saline and is injected through a catheter that is positioned within an antecubital vein. Patients will be equally randomized into any of the following treatment arms (for example, 0, 0.001, 0.005, 0.01, 0.025, 0.05, 0.10, 0.25, 0.5, and 1.0 mg/kg/hour). The peptide will be administered as an IV infusion from about 10 min prior to reperfusion to about 3 hours post-PCI. Following the reperfusion period, the subject may be administered the peptide chronically by any means of administration, *e*.*g*., subcutaneous or IV injection.

*Infarct Size.* The primary end point is the size of the infarct as assessed by measurements of cardiac biomarkers. Blood samples are obtained at admission and repeatedly over the next 3 days. Coronary biomarkers are measured in each patient. For example, the area under the curve (AUC) (expressed in arbitrary units) for creatine kinase and troponin I release (Beckman kit) may be measured in each patient by computerized planimetry. The principal secondary end point is the size of the infarct as measured by the area of delayed hyperenhancement that is seen on cardiac magnetic resonance imaging (MRI), assessed on day 5 after infarction. For the late-enhancement analysis, 0.2 mmol of gadolinium-tetrazacyclododecanetetraacetic acid (DOTA) per kilogram is injected at a rate of 4 ml per second and was flushed with 15 ml of saline. Delayed hyperenhancement is evaluated 10 min after the injection of gadolinium-DOTA with the use of a three-dimensional inversion-recovery gradient-echo sequence. The images are analyzed in shortaxis slices covering the entire left ventricle.

Myocardial infarction is identified by delayed hyperenhancement within the myocardium, defined quantitatively by an intensity of the myocardial postcontrast signal that is more than 2 SD above that in a reference region of remote, noninfarcted myocardium within the same slice. For all slices, the absolute mass of the infracted area is calculated according to the following formula: infarct mass (in grams of tissue) = ∑ (hyperenhanced area [in square centimeters]) × slice thickness (in centimeters) × myocardial specific density (1.05 g per cubic centimeter).

*Biomarkers to Established Risk Factors.* Levels of N-terminal pro-brain natriuretic peptide (NT-proBNP) and glucose, as well as estimated glomerular filtration rate (eGFR) are measured. These biomarkers all significantly predict all-cause mortality through a median follow-up of about two-and-a-half years. Calculating a risk score based on these three biomarkers can identify patients at high risk of dying during follow-up. It is predicted that the peptide will reduce the risk score of these biomarkers in patients undergoing PCI compared to patients undergoing PCI that do not receive the peptide. Blood samples may be taken for determination of the CK-MB and troponin I. The area under the curve (AUC) (expressed in arbitrary units) for CK-MB and troponin I release can be measured in each patient by computerized planimetry

*Other End Points.* The whole-blood concentration of peptide is immediately prior to PCI as well as at 1, 2, 4, 8 and 12 hours post PCI. Blood pressure and serum concentrations of creatinine and potassium are measured on admission and 24, 48, and 72 hours after PCI. Serum concentrations of bilirubin, γ-glutamyltransferase, and alkaline phosphatase, as well as white-cell counts, are measured on admission and 24 hours after PCI.

The cumulative incidence of major adverse events that occur within the first 48 hours after reperfusion are recorded, including death, heart failure, acute myocardial infarction, stroke, recurrent ischemia, the need for repeat revascularization, renal or hepatic insufficiency, vascular complications, and bleeding. The infarct-related adverse events are assessed, including heart failure and ventricular fibrillation. In addition, 3 months after acute myocardial infarction, cardiac events are recorded, and global left ventricular function is assessed by echocardiography (Vivid 7 systems; GE Vingmed).

It is predicted that administration of the peptide at the time of reperfusion will be associated with a smaller infarct by some measures than that seen with placebo.

### Example 2. Effects of Peptides in Providing Organ Protection During CABG

This Example will determine whether the administration of the aromatic-cationic peptide D-Arg-2'6'-Dmt-Lys-Phe-NH₂ ("the peptide") would limit the size of the injured myocardium in moderate to high-risk patients undergoing non-emergent CABG surgery with planned cardiopulmonary bypass (CPB) and cardioplegia. The effects of the peptide as a cardioprotective agent are evaluated using the relative size of injured myocardium as measured by peak CK-MB enzyme, toponin, or lactate dehydrogenase levels. The effects of administration of the peptide on renal and cardiac complications are also evaluated.

It is predicted that in conjunction with a background of standard-of-care therapy, the peptide is superior to placebo for the reduction of the incidence of cardiac, renal, and/or cerebral complications of elective CABG surgery with planned cardiopulmonary bypass and cardioplegia. The effects of the peptide as a cardioprotective agent as measured by the relative size of infarcted myocardium in moderate to high-risk patients undergoing elective CABG surgery with planned cardiopulmonary bypass and cardioplegia are measured by cardiac enzyme levels through post-operative day (POD) 4.

*Cardiac Complications.* This study has the following objectives: (1) To evaluate the effects of the peptide on the composite of cardiovascular death, nonfatal MI, or non-fatal stroke from randomization (post-operative day zero) through post-operative days (POD) 4, 30 and 90 in moderate to high-risk patients undergoing elective CABG surgery with planned cardiopulmonary bypass and cardioplegia; (2) To evaluate the effects of the peptide on the individual events of cardiovascular death, nonfatal MI, or non-fatal stroke from randomization (post-operative day zero) through post-operative days (POD) 4, 30 and 90 in moderate to high-risk patients undergoing elective CABG surgery with planned cardiopulmonary bypass and cardioplegia; and (3) To evaluate the incidence of important atrial and/or ventricular arrhythmias through POD 2 in moderate to high-risk patients undergoing elective CABG surgery with planned cardiopulmonary bypass and cardioplegia.

*Renal Complications.* This study has the following objectives: (1) To evaluate the effects of the peptide as a renal protective agent as measured by serial measurement of renal function for acute kidney injury (AKI) through POD 4 in moderate to high-risk patients undergoing elective CABG surgery with planned cardiopulmonary bypass and cardioplegia as measured by CK-MB levels through post-operative day (POD) 4; and (2) To evaluate the effects of the peptide on renal function from randomization (post-operative day zero) through post-operative day (POD) 30 and 90 in moderate to high-risk patients undergoing elective CABG surgery with planned cardiopulmonary bypass and cardioplegia.

*Cerebral Complications.* This study has the following objectives: (1) To evaluate the effects of the peptide on acute cerebral injury as assessed by magnetic resonance imaging performed prior to and by POD 4 (+2 days) in moderate to high-risk patients undergoing elective CABG surgery with planned cardiopulmonary bypass and cardioplegia; (2) To evaluate the safety and tolerability of a the peptide administered prior to, during, and for a short period of time after surgery in moderate to high-risk patients undergoing elective CABG surgery with planned cardiopulmonary bypass and cardioplegia; and (3) To evaluate the pharmacokinetics of the peptide in moderate to high-risk patients undergoing elective CABG surgery with planned cardiopulmonary bypass and cardioplegia.

### Overall Study Design and Plan

This study is a pilot phase II, prospective, randomized, double-blinded, placebo-controlled, multicenter dose-ranging study designed to test the hypothesis that the peptide in conjunction with a background of standard-of-care therapy is superior to placebo for the reduction of the incidence of cardiac, renal, and cerebral complications in moderate to high risk patients undergoing non-emergency CABG surgery with planned cardiopulmonary bypass (CPB) and cardioplegia. Surgical procedures include isolated coronary artery bypass graft (CABG) surgery with or without mitral valve repair for mild to moderate valvular dysfunction. Patients are considered moderate to high risk for subsequent end-organ complications associated with their CABG surgery. The patient's risk profile includes at least two of the following: age ≥ 65 years; moderate renal dysfunction defined as an estimated glomerular filtration rate (eGFR) 31 to 60 mL/min; history of diabetes mellitus requiring treatment other than diet; and evidence of significant left ventricular dysfunction (LV ejection fraction ≤0.40) or congestive heart failure without the presence of any type of cardiac pacemaker.

Major exclusion criteria include acute myocardial infarction occurring < 48 hours prior to randomization, CABG surgery using intermittent aortic cross clamping without cardioplegia, minimally invasive surgery (*i.e*., without use of CPB), clinically important renal and/or liver disease, uncontrolled diabetes, or history of a previous stroke, transient ischemic attack, or carotid endarterectomy as well as history of head trauma or seizures within the past six months. Prior to the planned CABG surgery patients will be screened for all inclusion/exclusion criteria. Qualified patients will be randomly assigned to receive one of two peptide dosing regimens or matching placebo in addition to standard treatment including anticoagulation.

The peptide or matching placebo will be administered *via* three different routes. All patients will receive study article in the same blinded manner using all three drug delivery modes: (1) as a systemic intravenous (IV) infusion commencing within approximately 30 minutes before induction of anesthesia and continuing for a total duration of approximately 6 hours, (2) in conjunction with the cardioplegia solution, and (3) as part of the priming solution in the heart lung machine during CPB.

In-hospital testing will continue for up to 96 hours. A diffusion weighted magnetic resonance imaging (DW-MRI) evaluation of the patient's brain will be performed within 4 days prior to and 3-6 days after the CABG surgery procedure. At a minimum, clinical follow-up for outcomes, laboratory data, and adverse events will be performed daily during the index hospitalization, and at 96 hours, 30 days (range, 30-40) and 90 days (range, 76-104) following the index CABG surgery procedure. Throughout the course of the trial, concomitant medical care will be left to the discretion of the cardiac surgeon and/or the caring physician. Adherence to guideline-based therapy and use of evidence-based medications (aspirin, β-blockers, angiotensin-converting-enzyme inhibitors, angiotensin II antagonists, calcium channel blockers, diuretics, anti-arrhythmic agents, statins, insulin, oral anti-diabetic drugs, and coumadin) will be strongly encouraged.

### Endpoints and Follow-up for Outcomes

The primary efficacy endpoint for the study will be an assessment of the effects of the peptide as a cardioprotective agent using the relative size of injured myocardium as measured by peak CK-MB enzyme levels through 72 hours post-operatively in moderate to high-risk patients undergoing non-emergent CABG surgery with planned cardiopulmonary bypass (CPB) and cardioplegia. Secondary endpoints will include the incidence of acute kidney injury (AKI) related to CABG surgery as assessed by serial measurements of renal function 72 hours post-operatively and the incidence of new acute cerebral injury related to CABG surgery as assessed at using magnetic resonance imaging of the brain on POD 3-6.

Pre-specified assessments for cardiovascular death, nonfatal MI, or non-fatal stroke will be performed from randomization (post-operative day zero) through POD 90. The diagnosis of MI will be based on clinical information collected from the study sites and CK-MB and electrocardiographic laboratory data from the core laboratories. Stroke will be defined as a new, focal, non-traumatic, neurological deficit lasting at least 24 hours. An independent, blinded clinical events committee will adjudicate all suspected MIs, strokes, and the cause of death for all deaths.

**Table 5: Schedule of Assessments- Initial 24 Hours**

| **Study Assessments** | **In Hospital Pre-Treatment Period** | **In Hospital Double-Blind Treatment Period** | | **In-Hospital Follow-up Period** |
|---|---|---|---|---|
| | **Screening** | **Cardiac Cath Lab** | **PCI To ∼3 Hrs Post PCI** | **∼3 to 24 Hrs Post PCI** |
| Medical history | X | | | |
| Medications on admission and prior to cardiac cath lab | X | | X | X |
| 12 lead ECG | Initial ECG | | | |
| Pregnancy test (urine) | X | | | |
| Review inclusion/exclusion criteria | X | | | |
| Informed consent | X | | | |
| Abbreviated Physical Exam | X | | | |
| Body weight | X | | | |
| Vital Signs (HR, BP, RR) | X | X | X | X |
| Serum creatinine, BUN, cystatin C | X | | | |
| Blood and urine chemistries | X | | | |
| Hematology (CBC with platelet count) | X | | | |
| CK-MB and Troponin I | X | Pre and post PCI | | Every 4 hours beginning at 4 hours post PCI |
| Serum NT pro-BNP | X | | | At 24 hours post PCI |
| hsCRP | X | | | At 24 hours post PCI |
| Diagnostic coronary angiogram | | X | | |
| TIMI assessment | | Pre and post PCI | | |
| Randomization¹ | | X | | |
| Study Drug Administration | | X | X | |
| Pharmacokinetic sampling | | Immediately pre PCI | 15, 60, 120, 180 minutes post PCI | 6, 12 and 24 hours post PCI |
| PCI and Stenting | | X | | |
| Review of ventricular ectopy | | X | X | X |
| NYHA Class | | | | X |
| Concomitant Medications | | | | |
| Adverse Event Reporting | | | | |
| SAE Reporting | | | X | X |

| | | | | |
|---|---|---|---|---|
| ¹ = Patients who do not fulfill per-protocol criteria for any reason (including pre and post-PCI TIMI flow criteria) will have study article discontinued and will be excluded from the efficacy analysis. These patients will be followed for safety for 72 hours and will be replaced in the randomization schema with a new patient. | | | | |

### Selection and Withdrawal of Patients

Subjects include male or female patients (age 45 or older) scheduled to undergo non-emergency CABG surgery with planned cardiopulmonary bypass (CPB) and cardioplegia. Surgical procedures will include isolated coronary artery bypass grafting (CABG) surgery with or without mitral valve repair for mild to moderate valvular dysfunction. Patients must be considered moderate to high risk for subsequent end-organ complications associated with their CABG surgery. The patient's risk profile must include at least two of the following: Age ≥ 65 years; moderate renal dysfunction defined as an estimated glomerular filtration rate (eGFR) 31 to 60 mL/min; History of diabetes mellitus requiring treatment other than diet; Evidence of significant left ventricular dysfunction (LV ejection fraction ≤0.40) or congestive heart failure without the presence of any type of cardiac pacemaker.

### Treatment of Patients

*Study Drug.* D-Arg-2'6'-Dmt-Lys-Phe-NH₂ is a small peptide (CAS No. 736992-21-5). Its molecular weight is 639.8 (free base). It is stable to light in either powder or liquid form. It is stable up to 40°C and resistant to oxidation. The drug substance will be provided as a lyophilized powder in sterile glass vials. Each vial will be reconstituted with 10 mL of sterile D5W by the unblinded pharmacist at each site.

The study will be conducted in a blinded manner at the study site (patients and site personnel will be blinded). The randomization code will be created by an independent statistician. Patients will be equally randomized into any of the following treatment arms (0, 0.001, 0.005, 0.01, 0.025, 0.05, 0.10, 0.25, 0.50, or 1 mg/kg/hour). The reconstituted, diluted study drug will be infused at 60 mL/hr using an infusion pump and started at least 10 minutes prior to the anticipated reperfusion time and continuing for 3 hours after the CABG procedure. Start and completion times will be recorded for intravenously administered study medication. The volume of study solution left in the infusion bag will be recorded (estimation by eye is sufficient) and provide a check that the proper amount of diluted study material was infused. The plasma level of the peptide will be measured and will provide the most accurate measure of treatment compliance.

### Assessment of Efficacy

The primary analysis for efficacy will be a comparison of the left ventricular infarct size estimated by the area under the curve (AUC) for the creatine kinase-MB curve through 72 hours among the placebo and each of the peptide dose groups. Secondary efficacy analyses will focus on the effects of the peptide on myocardial injury as measured by: (1) area under the troponin I enzyme curve over 72 hours; (2) cardiac magnetic resonance imaging (CMR) at 4 ±1 days, 30 ± 3 days and 6 + 1.5 months; (3) incidence of post-reperfusion arrhythmias; and (4) microvascular obstruction. These analyses will be performed for patients who have baseline TIMI flow grade = 0, TIMI flow grade = 1, and all patients (TIMI flow grade of either 0 or 1.

The immediate benefits after CABG will be examined, including: (1) Degree of coronary arterial flow, and incidence of arrhythmias; (2) The 30 day and 6 month effects of the peptide on myocardial function and remodeling in patients as measured by CMR will be determined; (3) The effect of the peptide on the incidence of microvascular obstruction; (4) The pharmacokinetics of the peptide in patients who have undergone successful reperfusion; and (5) The immediate, 30 day, 90 day and 6 month effects of the peptide on renal function as measured by serum creatinine, estimated creatinine clearance, cystatin C, and BUN.

*Cardiac Biomarkers.* Blood samples are taken for determination of the CK-MB and troponin I. The area under the curve (AUC) (expressed in arbitrary units) for CK-MB and troponin I release will be measured in each patient by computerized planimetry at the following timepoints: on admission; before and after CABG, through the sheath; every 4 hours after CABG during the first 24 hours; every 6 hours after CABG during the second and third day; and after the third day, as clinically indicated. Blood samples for determination of NT-proBNP and CRP levels will be taken at the following timepoints: pre-CABG; at 24 hours after CABG; at 30 ± 3 days after CABG; at 90 ± 14 days after CABG; and at 6 + 1.5 months after CABG.

*Cardiac MR Imaging (CMR).* A 1.5-T body MRI scanner will be used to perform CMR in order to assess ventricular function, myocardial edema (area at risk), microvascular obstruction and infarct size. CMR will be performed at 4 ± 1 days, 30 ± 3 days and 6 + 1.5 months after successful CABG. The specific CMR protocol includes taking cine images for left ventricular volumes, mass and ejection fraction. Cine imaging techniques with steady-state free precession sequences will be performed at day 4 ± 1, day 30 ± 3 and 6 + 1.5 months after successful CABG. T2-weighted images will be taken to assess myocardial edema for determination of ischemic area-at-risk for infarction. A triple inversion recovery fast spin echo sequences will be performed only at the Day 4 ± 1 CMR study. Post-contrast delayed enhancement will be used on day 4 ± 1, day 30 ± 3 and 6 + 1.5 months after surgery to quantify infracted myocardium. This will be defined quantitatively by an intensity of the myocardial post-contrast signal that is more than 2 SD above that in a reference region of remote, non-infarcted myocardium within the same slice. Standard extracellular gadolinium-based contrast agents will be used at a dose of 0.2 mmol/kg. 2D inversion recovery prepared fast gradient echo sequences will be used at the following time points: (1) Early (approximately 2 minutes after contrast injection) for evaluation of microvascular obstruction. Single shot techniques may be considered if available; and (2) Late (approximately 10 minutes after contrast injection) for evaluation of infarct size.

Blood pressure, heart rate, and respiratory rate will be serially measured throughout the trial. Blood and urine chemistries as well as hematology profiles will be serially measured during the trial and will include: electrolytes (sodium, potassium, bicarbonate, chloride); liver function (total bilirubin, aspartate aminotransferase [AST or SGOT] and alanine aminotransferase [ALT or SGPT]); kidney function (Serum creatinine, cystatin C, and blood urea nitrogen [BUN]); estimated glomerular filtration rate (eGFR); incidence of acute kidney injury (AKI) post CABG surgery; and complete blood count.

Renal function will be assessed by serial measurements of serum creatinine and cystatin C, and BUN; serial calculations of estimated glomerular filtration rate (eGFR); and incidence of at least a grade 1 episode of contrast-induced nephropathy post CABG defined as an increase in serum creatinine of ≥ 25% of the baseline value and/or an increase in serum creatinine of 0.5 mg/dl occurring within 48 hours of receiving a radiographic contrast agent.

*Biomarkers to Established Risk Factors.* Levels of N-terminal pro-brain natriuretic peptide (NT-proBNP) and glucose, as well as estimated glomerular filtration rate (eGFR) are measured. These biomarkers all significantly predict all-cause mortality through a median follow-up of about two-and-a-half years. Calculating a risk score based on these three biomarkers can identify patients at high risk of dying during follow-up. It is predicted that the peptide will reduce the risk score of these biomarkers in patients undergoing CABG compared to patients undergoing CABG that do not receive the peptide.

### Predicted Outcomes

It is predicted that the peptide will reduce infarct size and reduce the incidence of renal AKI and cerebral complications relative to subjects undergoing CABG, but who do not receive the peptide. The primary analysis for efficacy will be a comparison of the LV infarct size estimated by the area under the curve (AUC) for the creatine kinase-MB curve through 72 hours among the placebo and each of the two peptide dose groups using an ANOVA model (or the non-parametric equivalent, the Kruskal-Wallis analysis of ranks, if the distribution is judged to be non-Gaussian). It is also predicted that the peptide will act as a multi-organ protectant when administered prior to, during, and immediately post-surgery in patients who are undergoing a CABG procedure with planned cardiopulmonary bypass and cardioplegia.

### References

1. ACC/AHA 2004 Guideline Update for Coronary Artery Bypass Graft Surgery Circulation 2004;110:e340-e437
2. Sellke FW, DiMaio JM, Caplan LR, et. al. Comparing On-Pump and Off-Pump Coronary Artery Bypass Grafting: A Scientific Statement From the American Heart Association Council on Cardiovascular Surgery and Anesthesia in Collaboration With the Interdisciplinary Working Group on Quality of Care and Outcomes Research. Circulation 2005;111;2858-2864.
3. Heart Disease and Stroke Statistics: 2010 Update At-A-Glance. American Heart Association, Dallas, Texas, 2010.
4. Shroyer AL, Grover FL, Hattler B. On-Pump versus Off-Pump Coronary-Artery Bypass Surgery. N Engl J Med 2009;361:1827-37.
5. Crescenzi G, Landoni G, Bignami E, et. al. N-Terminal B-Natriuretic Peptide After Coronary Artery Bypass Graft Surgery. J Cardiothor Vasc Anesth 2009;23:147-150
6. Costa MA, Carere RG, Lichtenstein SV, et al. Incidence, predictors, and significance of abnormal cardiac enzyme rise in patients treated with bypass surgery in the Arterial Revascularization Therapies
7. Study (ARTS). Circulation. 2001;104(22):2689-2693.
8. Klatte K, Chaitman BR, Theroux P, et al. Increased mortality after coronary artery bypass graft surgery is associated with increased levels of post-operative creatine kinase-myocardial band isoenzyme release: results from the GUARDIAN trial. J Am Coll Cardiol. 2001;38(4):1070-1077.
9. Thygesen K, Alpert JS, White HD. Universal definition of myocardial infarction. Eur Heart J. 2007; 28(20):2525-2538.
10. Makhija N, Sendasgupta C, Kiran U, et. al. The role of oral coenzyme Q10 in patients undergoing coronary artery bypass graft surgery. J Cardiothor Vasc Anesth 2008;22:832-9.
11. Ochoa JJ, Vilchez MJ, Ibanez S, et al: Oxidative stress is evident in erythrocytes as well as plasma in patients undergoing heart surgery involving cardiopulmonary bypass. Free Radic Res 37:11-17, 2003.
12. Hammond B, Hess ML: The oxygen free radical system: Potential mediator of myocardial injury. J Am Coll Cardiol 6:215-220, 1985.
13. Shlafer M, Kane PF, Kirsh M: Superoxide dismutase plus catalase enhances the efficacy of hypothermic cardioplegia to protect the globally ischemic, reperfused heart. J Thorac Cardiovasc Surg 83:830-839, 1982.
14. Menasche P, Grousset C, Gauduel Y, et al: A comparative study of free radical scavengers in cardioplegic solutions. Improved protection with peroxidase. J Thorac Cardiovasc Surg 92:264-271, 1986.
15. Pechan I, Olejarova J, Danova K, et al: Antioxidant status of patients after on-pump and off-pump coronary artery bypass grafting. Bratisl Lek Listy 105:45-50, 2004
16. Bolli R, Becker L, Gross G, et. al. Myocardial protection at a crossroads: The need for translation into clinincal therapy. Circ Res. 2004. 95: p. 125-34.
17. Hausenloy, D.J. and D.M. Yellon, Time to take myocardial reperfusion injury seriously. N Engl J Med, 2008. 359: p. 518-20.
18. Hausenloy, D.J. and D.M. Yellon, Preconditioning and postconditioning: united at reperfusion. Pharmacol Ther, 2007. 116: p. 173-91.
19. Ruiz-Meana, M. and D. Garcia-Dorado, Translational cardiovascular medicine (II). Pathophysiology of ischemia-reperfusion injury: new therapeutic options for acute myocardial infarction. Rev Esp Cardiol, 2009. 62: p. 199-209.
20. Verma, S., et al., Fundamentals of reperfusion injury for the clinical cardiologist. Circulation, 2002. 105: p. 2332-6.
21. Piot, C., et al., Effect of cyclosporine on reperfusion injury in acute myocardial infarction. N Engl J Med, 2008. 359: p. 473-81.
22. Hausenloy, D.J., M.R. Duchen, and D.M. Yellon, Inhibiting mitochondrial permeability transition pore opening at reperfusion protects against ischaemia-reperfusion injury. Cardiovasc Res, 2003. 60: p. 617-25.
23. Morin D, Assaly R, Paradis S, Berdeaux A. Inhibition of mitochondrial membrane permeability as a putative pharmacological target for cardioprotection. Cur Med Chemistry. 2009. 16: p. 4382-98.
24. Akar, F.G., et al., The mitochondrial origin of postischemic arrhythmias. J Clin Invest, 2005. 115: p. 3527-35.
25. Yellon DM, Hausenloy DJ. Myocardial reperfusion injury. N Engl J Med. 2007;357(11):1121-1135.
26. Verrier ED, Sherman SK, Taylor KM, et al. Terminal complement blockade with pexelizumab during coronary artery bypass graft surgery requiring cardiopulmonary bypass. JAMA. 2004;291(19):2319- 2327.
27. Shernan SK, Fitch JC, Nussmeier NA, et al. Impact of pexelizumab, an anti-C5 complement antibody, on total mortality and adverse cardiovascular outcomes in cardiac surgical patients undergoing cardiopulmonary bypass. Ann Thorac Surg. 2004;77 (3):942-950.
28. Smith PK, Carrier M, Chen JC, et al. Effect of pexelizumab in coronary artery bypass graft surgery with extended aortic cross-clamp time. Ann Thorac Surg 2006;82:781-9.
29. Carrier M, Menasche P, Levy JH, et al. Inhibition of complement activation by pexelizumab reduces death in patients undergoing combined aortic valve replacement and coronary artery bypass surgery. Ann Thorac Surg 2006;131:352-6.
30. Zhao, K., et al., Mitochondria-targeted peptide prevents mitochondrial depolarization and apoptosis induced by tert-butyl hydroperoxide in neuronal cell lines. Biochem Pharmacol, 2005;70:1796-806.
31. Szeto H. Mitochondria-targeted cytoprotective peptides for ischemia-reperfusion injury. Antioxidants & Redox Signaling. 2008. 10: p. 601-19.
32. Zhao, K., et al., Cell-permeable peptide antioxidants targeted to inner mitochondrial membrane inhibit mitochondrial swelling, oxidative cell death, and reperfusion injury. J Biol Chem, 2004;279:34682-90.
33. Whiteman, M., et al., Do mitochondriotropic antioxidants prevent chlorinative stress-induced mitochondrial and cellular injury? Antioxid Redox Signal, 2008;10:641-50.
34. Yang, L., et al., Mitochondria targeted peptides protect against 1-methyl-4-phenyl-1,2,3,6-tetrahydropyridine neurotoxicity. Antioxid Redox Signal, 2009;11:2095-104.
35. Cho, J., et al., Potent mitochondria-targeted peptides reduce myocardial infarction in rats. Coron Artery Dis, 2007. 18(3): p. 215-20.
36. Leshnower BG, Kanemoto S, Matsubara M, et. al. Cyclosporine preserves mitochondrial morphology after myocardial ischemia/reperfusion independent of calcineurin inhibition. Ann Thorac Surg. 2008. 86: 1286-92.
37. Mewton N, Croisille P, Gahide G, et. al. Effect of cyclosporine on left ventricular remodeling after reperfused myocardial infarction. J Am Coll Cardiol, 2010;55:1200-5.
38. Lassnigg A, Schmid ER, Hiesmayr M, et al: Impact of minimal increases in serum creatinine on outcome in patients after cardiothoracic surgery: Do we have to revise current definitions of acute renal failure? Crit Care Med 36:1129-1137, 2008
39. Lassnigg A, Schmidlin D, Mouhieddine M, et al: Minimal changes of serum creatinine predict prognosis in patients after cardiothoracic surgery: A prospective cohort study. J Am Soc Nephrol 15:1597-1605, 2004
40. Devbhandari MP, Duncan AJ, Grayson AD, et al: Effect of risk-adjusted, non-dialysis-dependent renal dysfunction on mortality and morbidity following coronary artery bypass surgery: A multi-centre study. Eur J Cardiothorac Surg 29:964-970, 2006
41. Weerasinghe A, Hornick P, Smith P, et al: Coronary artery bypass grafting in non-dialysis-dependent mild-to-moderate renal dysfunction. J Thorac Cardiovasc Surg 121:1083-1089,2001
42. Brown JR, Cochran RP, MacKenzie TA, et al: Long-term survival after cardiac surgery is predicted by estimated glomerular filtration rate. Ann Thorac Surg 86:4-11, 2008
43. Zakeri R, Freemantle N, Barnett V, et al: Relation between mild renal dysfunction and outcomes after coronary artery bypass grafting. Circulation 112:I270-I275, 2005
44. Karkouti K; Wijeysundera DN, Yau TM, et.al. Acute kidney injury after cardiac surgery - Focus on modifiable risk factors. Circulation. 2009;119:495-502
45. Wijeysundera DN, Karkouti K, Dupuis JY, et. al. Derivation and validation of a simplified predictive index for renal replacement therapy after cardiac surgery. JAMA. 2007;297:1801-9.
46. Kuitunen A, Vento A, Suojaranta-Ylinen R, et al: Acute renal failure after cardiac surgery: Evaluation of the RIFLE classification. Ann Thorac Surg 81:542-546, 2006
47. Maslow AD, Chaudrey A, Bert A. Schwartz C, Singh A. Peri-operative renal outcome in cardiac surgical patients with preoperative renal dysfunction: Aprotinin versus epsilon aminocaproic acid. J Cardiothor and Vasc Anesth 2008;22:6-15.
48. Mangano DT, Tudor IC, Dietzel C, for the Multicenter Study of Peri-operative Ischemia Research Group and the Ischemia Research and Education Foundation: The risk associated with aprotinin in cardiac surgery. N Engl J Med 354:353-365, 2006
49. Anderson RJ, O'Brien M, Mawhinney S, et al: Renal failure predisposes patients to adverse outcome after coronary artery bypass surgery. Kidney Int 55:1057-1062, 1999
50. Nakayama Y, Sakata R, Ura M, et al: Long-term results of coronary artery bypass grafting in patients with renal insufficiency. Ann Thorac Surg 75:496-500, 2003
51. Penta de Peppo A, Nardi P, De Paulis R, et al: Cardiac surgery in moderate-to-end-stage renal failure: Analysis of risk factors. Ann Thorac Surg 74:378-383, 2002
52. Durmaz L, Buket S, Atay Y, et al: Cardiac surgery with cardiopulmonary bypass in patients with chronic renal failure. J Thorac Cardiovasc Surg 118:306-315, 1999
53. Durmaz I, yagdi T, Clkavur T, et. al. Prophylactic dialysis in patients with reanl dysfunction undergoing on-pump coroanary bypass surery. Ann Thorac Surg 2003;75:859-64.
54. Bellomo R, Ronco C, Kellum JA, et al, and the ADQI Workgroup: Acute renal failure-Definition, outcome measures, animal models, fluid therapy and information technology needs: The Second International Consensus Conference of the Acute Dialysis Quality Initiative (ADQI) Group. Available at: http://ccforum.com/content/8/4/R204.
55. Mehta RL, Kellum JA, Shah SV, et al: Acute Kidney InjuryNetwork: Report of an initiative to improve outcomes in acute kidney injury. Crit Care 11:R31, 2007
56. Garwood S. Cardiac surgery-associated acute renal injury: new paradigms and innovative therapies. Journal of Cardiothoracic and Vascular Anesthesia, 2010; In Press.
57. Haase M, Bellomo R, Matalanis G, et al: A comparison of the RIFLE and Acute Kidney Injury Network classifications for cardiac surgery-associated acute kidney injury: A prospective cohort study. J Thorac Cardiovasc Surg 138:1370-1376, 2009.
58. Abuelo JG. Normotensive ischemic acute renal failure. N Engl J Med 2007;357:797-805.
59. Fontaine D, Pradier O, Hacquebard M, et al: Oxidative stress produced by circulating microparticles in on-pump but not off pump coronary surgery. Acta Cardiol 2009;64:715-22.
60. Gerritsen WB, van Boven WJ, Driessen AH, et al: Off-pump versus on-pump coronary artery bypass grafting: Oxidative stress and renal function. Eur J Cardiothorac Surg 2001;20:923-9.
61. Saikumar P, Venkatachalam MA: Role of apoptosis in hypoxic/ischemic damage to the kidney. Semin Nephrol 2003;23:511-21.
62. Kaushal GP, Basnakian AG, Shah SV: Apoptotic pathways in ischemic acute renal failure. Kidney Int 2004;66:500-6.
63. Dagher PC: Apoptosis in ischemic renal injury: Roles of GTP depletion and p53. Kidney Int 2004;66:5006-9.
64. Castaneda MP, Swiatecka-Urban A, Mitsnefes MM, et al: Activation of mitochondrial apoptotic pathways in human renal allografts after ischemia-reperfusion injury. Transplantion 2003;76:500-4.
65. Granville DJ, Shaw JR, Leong S, et al: Release of cytochrome-c, Bax migration, Bid cleavage, and activation of caspases 2,3,6,7,8, and 9 during endothelial apoptosis. Am J Pathol 1999;155:1021-5.
66. Kelly KJ, Plotkin Z, Vulgamott SL, et al: P53 mediates theapoptotic responses to GTp depletion after renal ischemia-reperfusion Protective role of a p53 inhibitor. J Am Soci Nephol 203;14:128-38.
67. Molitoris BA, Sutton TA: Endothelial injury and dysfunction: Role in the extension phase of acute renal failure, Kidney Int 2004;66:496-9.
68. Bonventre JV, Zuk A: Ischemic acute renal failure: An inflammatory disease? Kidney Int 2004;66:480-5.
69. Friedewald JJ, Rabb H: Inflammatory cells in ischemic acute renal failure. Kidney Int 2004;66:486-91.
70. Laffey JG, Boylan JF, Cheng DCH. The systemic inflammaory response to cardiac surgery. Anesthesiology. 2002;97:215-252.
71. Hudetz JA, Pagel PS. Neuroprotection by Ketamine: A Review of the Experimental and Clinical Evidence. J Cardiothorac Vasc Anesth. 2010;24:131-142.
72. Harrison MJ. Neurologic complications of coronary artery bypass grafting: diffuse or focal ischemia? Ann Thorac Surg 1995;59:1356-8.
73. Hornick P, Smith PL, Taylor KM. Cerebral complications after coronary bypass grafting. Curr Opin Cardiol 1994;9:670-9.
74. Abe T, Shimamura M, Jackman K, et. al. Key Role of CD36 in Toll-Like Receptor 2 Signaling in Cerebral Ischemia. Stroke 2010;41:898-904.
75. Cho S, Park M, Febbraio M, et. al. The Class B Scavenger Receptor CD36 Mediates Free Radical Production and Tissue Injury in Cerebral Ischemia. J Neruroscience 2005;25:2504-12.
76. Lakhan SE, Kirchgessner A, Hofer M. Inflammatory mechanisms in ischemic stroke: therapeutic approaches. J Translational Med 2009;7:97-104.
77. Cho S, Kim E. CD36: A multi-modal target for acute stroke therapy. J Neurochem 2009;109 (Suppl 1): 126-32.
78. Cook DJ, Huston J, Trenerry MR, et. al. Postcardiac Surgical Cognitive Impairment in the Aged Using Diffusion-Weighted Magnetic Resonance Imaging. Ann Thorac Surg. 2007;83:1389-1395
79. van Everdingen KJ, van der Grond J, Kappelle LJ, et. al. Diffusion-weighted magnetic resonance imaging in acute stroke. Stroke 1998;29:1783-90.
80. Nakamura H, Yamada K, Kizu O, et al. Effect of thin-section diffusion-weighted MR imaging on stroke diagnosis. AJNR Am J Neuroradiol 2005;26:560-5.
81. Mullins ME, Schaefer PW, Sorensen AG, et al. CT and conventional and diffusion-weighted MR imaging in acute stroke: study in 691 patients at presentation to the emergency department. Radiology 2002;224:353-60.
82. Maekawa K, Goto T, Baba T, et. al. Abnormalities in the Brain Before Elective Cardiac Surgery Detected by Diffusion-Weighted Magnetic Resonance Imaging. Ann Thorac Surg 2008;86:1563-9
83. Knipp SC, Matatko N, Wilhelm H, et. al. Cognitive Outcomes Three Years After Coronary Artery Bypass Surgery: Relation to Diffusion-Weighted Magnetic Resonance Imaging. Ann Thorac Surg 2008;85:872-879
84. Van Dijk D, Jansen EW, Hijman R, et al. Cognitive outcome after off-pump and on-pump coronary artery bypass graft surgery: a randomized trial. JAMA 2002;287:1405-12.
85. Kong RS, Butterworth J, Aveling W, et al. Clinical trial of the neuroprotectant clomethiazole in coronary artery bypass graft surgery: a randomized controlled trial. Anesthesiology 2002;97:585-91.
86. Butterworth J, Wagenknecht LE, Legault C, et al. Attempted control of hyperglycemia during cardiopulmonary bypass fails to improve neurologic or neurobehavioral outcomes in patients without diabetes mellitus undergoing coronary artery bypass grafting. J Thorac Cardiovasc Surg 2005;130:1319.
87. Grigore AM, Mathew J, Grocott HP, et al. Prospective randomized trial of normothermic versus hypothermic cardiopulmonary bypass on cognitive function after coronary artery bypass graft surgery. Anesthesiology 2001;95:1110-9.
88. Seines OA, Zeger SL. Optional Coronary Artery Bypass Grafting Baseline Cognitive Assessment: Essential not optional. Ann Thorac Surg 2007;83:374-6.
89. Marasco SF, Sharwood LN, Abramson MJ. No improvement in neurocognitive outcomes after off-pump versus on-pump coronary revascularisation: a meta-analysis. Eur J Cardiothorac Surg 2008;33:961-970.
90. Rasmussen LS, Johnson T, Kuipers HM, et al. Does anaesthesia cause postoperative cognitive dyfunction? A randomized study of regional versus general anaesthesia in 438 elderly patients. Acta Anaesthesiol Scand 2003;47:260-6.
91. Wahrborg P, Booth JE, Clayton T, et al. Neuropsychological outcome after percutaneous coronary inetrvention or coronary artery bypass grafting: results from the Stent or Surgery (SoS) trial. Circulation 2004;110:3411-7.
92. Jensen BO, Hughes P, Rasmussen LS, et al. Cognitive outcomes in elderly high-risk patients after off-pump versus conventional coronary artery bypass grafting. A randomized trial. Circulation 2006;113:2790-5.
93. Cho S, Szeto HH, Kim E, et. al. A Novel Cell-permeable Antioxidant Peptide, SS31, Attenuates Ischemic Brain Injury by Down-regulating CD36. J Biological Chem 2007;282:4634-42.

### EQUIVALENTS

The present invention is not to be limited in terms of the particular embodiments described in this application, which are intended as single illustrations of individual aspects of the invention. Many modifications and variations of this invention can be made without departing from its spirit and scope, as will be apparent to those skilled in the art. Functionally equivalent methods and apparatuses within the scope of the invention, in addition to those enumerated herein, will be apparent to those skilled in the art from the foregoing descriptions. Such modifications and variations are intended to fall within the scope of the appended claims. The present invention is to be limited only by the terms of the appended claims, along with the full scope of equivalents to which such claims are entitled. It is to be understood that this invention is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc*. As a non-limiting example, each range discussed herein can be readily broken down into a lower third, middle third and upper third, *etc*. As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, provisional applications, and publications referred to or cited herein are incorporated by reference in their entirety, including all figures and tables, to the extent they are not inconsistent with the explicit teachings of this specification. Other embodiments are set forth within the following claims.

## Claims

1. A composition comprising the peptide D-Arg-2'6'-Dmt-Lys-Phe-NH₂ or a pharmaceutically acceptable salt thereof for use in treating obstructive coronary artery disease comprising administering an effective amount of the peptide to a subject, wherein the subject undergoes a coronary artery bypass graft (CABG) procedure.

2. The composition for use according to claim 1, for administration prior to the coronary artery bypass graft (CABG) procedure.

3. The composition for use according to claim 1, for administration after the coronary artery bypass graft procedure (CABG) procedure.

4. The composition for use according to claim 1, for administration during and after the coronary artery bypass graft (CABG) procedure.

5. The composition for use according to claim 1, for administration continuously before, during, and after the coronary artery bypass graft (CABG) procedure.

6. The composition for use according to claim 5, for administration for at least 3 hours after the coronary artery bypass graft (CABG) procedure; or
for administration for at least 5 hours after the coronary artery bypass graft (CABG) procedure; or
for administration for at least 8 hours after the coronary artery bypass graft (CABG) procedure; or
for administration for at least 12 hours after the coronary artery bypass graft (CABG) procedure; or
for administration for at least 24 hours after the CABG the coronary artery bypass graft (CABG) procedure.

7. The composition for use according to claim 5, for administration starting at least 8 hours before the coronary artery bypass graft (CABG) procedure; or
for administration starting at least 5 hours before the coronary artery bypass graft (CABG) procedure; or
for administration starting at least 2 hours before the coronary artery bypass graft (CABG) procedure; or
for administration starting at least 1 hour before the coronary artery bypass graft (CABG) procedure; or
for administration starting at least 30 minutes before the coronary artery bypass graft (CABG) procedure.

8. The composition for use according to claim 1, wherein the composition is for administration by a systemic intravenous infusion commencing about 30 minutes before an induction of anesthesia.

9. The composition for use according to claim 1, wherein the composition is for administration in conjunction with a cardioplegia solution.

10. The composition for use according to claim 1, wherein the composition is for administration as part of the priming solution in a heart lung machine during the CABG procedure.

11. The composition for use according to claim 1, wherein the levels of one or more of N-terminal pro-brain natriuretic peptide (NT-proBNP), glucose, and estimated glomerular filtration rate (eGFR) are reduced in a subject administered the composition relative to a comparable subject undergoing the CABG procedure, but not administered the composition.

12. A composition comprising the peptide D-Arg-2'6'-Dmt-Lys-Phe-NH₂ or a pharmaceutically acceptable salt thereof for use in preventing renal or cerebral complications in a subject, wherein the subject undergoes a coronary artery bypass graft (CABG) procedure.
